# EUROPEAN PATENT APPLICATION

(11) **EP 4 089 110 A1**
(43) Date of publication of application: **16.11.2022**
(21) Application number: 20911527.8
(22) Date of filing: 28.12.2020
(51) Int. Cl.: C07K 16/00, C12P 1/00, C12P 21/00

(54) **ANTIBODY POPULATION UNIFORMLY INCLUDING ANTIBODIES HAVING LEFT-RIGHT ASYMMETRIC SUGAR CHAIN, AND METHOD FOR PRODUCING SAME**

(30) Priority: 10.01.2020 JP 2020002968
(71) Applicant: RIKEN, Wako-shi, Saitama 351-0198 (JP); Fushimi Pharmaceutical Co., Ltd., Marugame-shi, Kagawa 763-8605 (JP)
(72) Inventor: MANABE Shino, Wako-shi, Saitama 351-0198 (JP); YAMAGUCHI Yoshiki, Wako-shi, Saitama 351-0198 (JP); IWAMOTO Shogo, Marugame-shi, Kagawa 763-8605 (JP); KINOSHITA Takashi, Marugame-shi, Kagawa 763-8605 (JP)
(74) Representative: Altmann Stößel Dick Patentanwälte PartG mbB
(86) International application number: PCT/JP2020/049120
(87) International publication number: WO 2021/140981

(57) **Abstract**

Provided are a method for preparing a population of antibodies comprising homogeneous antibodies each having left-right asymmetric sugar chains, and a population of homogeneous antibodies each having left-right asymmetric sugar chains, obtained by the method. The present invention provides a method for producing a population of antibodies comprising homogeneous antibodies in which N-linked complex sugar chains attached to asparagine (Asn) at position 297 of CH domains of Fc regions of two heavy chains on the left and the right of each antibody are sugar chains structurally different from each other, the method comprising the steps of: (i) cleaving sugar chains attached to two heavy chains on the left and the right of each antibody in an antibody composition with endo-β-N-acetylglucosaminidase (ENGase), and purifying and isolating antibodies from which the sugar chains of both the heavy chains have been cleaved; (ii) mixing a population of the antibodies from which the sugar chains of both the heavy chains have been cleaved, obtained in the step (i), any sugar chain X in an oxazoline form or an oxazoline-generating sugar derivative, the sugar chain of which is sugar chain X, and ENGase modified so as to suppress sugar chain cleavage activity and improve sugar chain transfer activity, to prepare antibodies in which the sugar chain X is attached to only one of the two heavy chains on the left and the right, followed by purification and isolation of the antibodies; and (iii) mixing the antibodies in which the sugar chain X is attached to only one of the two heavy chains on the left and the right, obtained in the step (ii), sugar chain Y in an oxazoline form which is structurally different from the sugar chain X, or an oxazoline-generating sugar derivative, the sugar chain of which is sugar chain Y, and ENGase modified so as to suppress sugar chain cleavage activity and improve sugar chain transfer activity, to prepare antibodies in which the sugar chain Y is attached to the other heavy chain of the two heavy chains on the left and the right and in which the sugar chains attached to the two heavy chains on the left and the right are sugar chains structurally different from each other, followed by purification and isolation of the antibodies.

## Description

### Technical Field

The present invention relates to a population of antibodies having sugar chain structures homogeneous among the antibodies, with each antibody having sugar chains on the left and the right structurally different from each other, and a method for producing the same.

### Background Art

Antibody sugar chains are heterogeneous and are mixtures having slightly different sugar chain structures. The antibody sugar chains are known to influence effector functions such as ADCC (antibody dependent cellular cytotoxicity) and antibody functions such as immunogenicity. In order to prepare more highly functional antibodies, the modification of sugar chain structures has received attention. At this moment, it is impossible to isolate antibodies having homogeneous sugar chain structures from antibodies differing in sugar chain structure prepared in CHO (Chinese hamster ovary) cells, because of a repertoire of microstructurally different sugar chain structures and very similar physicochemical properties. Such sugar chains have been modified for homogeneity using endo-β-N-acetylglucosaminidase (ENGase) and modified ENGase to prepare antibodies having left-right symmetric sugar chain structures homogeneous among the antibodies. Specifically, antibodies having homogeneous sugar chain structures have been prepared by the cleavage of structurally heterogeneous sugar chains with ENGase and subsequent glycosylation using synthesized or isolated sugar chains and modified ENGase (Patent Literature 1). The previous approach merely synthesizes antibodies each having left-right symmetric sugar chains.

### Citation List

### Patent Literature

Patent Literature 1: International Publication No. WO2013/120066

### Summary of Invention

### Technical Problem

An object of the present invention is to provide a method for preparing a population of antibodies comprising homogeneous antibodies each having left-right asymmetric sugar chains, and a population of homogeneous antibodies each having left-right asymmetric sugar chains, obtained by the method.

### Solution to Problem

The present inventors have completed the present invention by finding that antibodies, each of which has sugar chains structurally different from each other and which have sugar chain structures on the left and the right homogeneous among the antibodies, can be obtained by appropriately adjusting the amount of sugar chain-oxazoline X (sugar chain X in an oxazoline form) for glycosylation reaction, and isolating antibodies each glycosylated at only one heavy chain using a column with FcγRIIIa immobilized thereon, followed by reaction with sugar chain-oxazoline Y (sugar chain Y in an oxazoline form) structurally different therefrom. No technique of preparing a population of structurally homogeneous antibodies each having sugar chains on the left and the right structurally different from each other has been known so far.

Specifically, the present invention is as follows.
[1] A population of antibodies comprising homogeneous antibodies in which N-linked complex sugar chains attached to asparagine (Asn) at position 297 of CH domains of Fc regions of two heavy chains on the left and the right of each antibody are sugar chains structurally different from each other.
[2] The population of antibodies according to [1], wherein the population comprises 90% or more of the antibodies in which the N-linked complex sugar chains attached to asparagine (Asn) at position 297 of CH domains of Fc regions of two heavy chains on the left and the right of each antibody are different from each other.
[3] The population of antibodies according to [1] or [2], wherein N-acetylglucosamine (GlcNAc) at reducing ends of the N-linked complex sugar chains attached to asparagine (Asn) at position 297 located in the CH domains of the antibody Fc regions is fucosylated.
[4] A method for producing a population of antibodies comprising homogeneous antibodies in which N-linked complex sugar chains attached to asparagine (Asn) at position 297 of CH domains of Fc regions of two heavy chains on the left and the right of each antibody are sugar chains structurally different from each other, the method comprising the steps of:
   (i) cleaving sugar chains attached to two heavy chains on the left and the right of each antibody in an antibody composition with endo-β-N-acetylglucosaminidase (ENGase), and purifying and isolating antibodies from which the sugar chains of both the heavy chains have been cleaved;
   (ii) mixing a population of the antibodies from which the sugar chains of both the heavy chains have been cleaved, obtained in the step (i), any sugar chain X in an oxazoline form or an oxazoline-generating sugar derivative, the sugar chain of which is sugar chain X, and ENGase modified so as to suppress sugar chain cleavage activity and improve sugar chain transfer activity, to prepare antibodies in which the sugar chain X is attached to only one of the two heavy chains on the left and the right, followed by purification and isolation of the antibodies; and
   (iii) mixing the antibodies in which the sugar chain X is attached to only one of the two heavy chains on the left and the right, obtained in the step (ii), sugar chain Y in an oxazoline form which is structurally different from the sugar chain X, or an oxazoline-generating sugar derivative, the sugar chain of which is sugar chain Y, and ENGase modified so as to suppress sugar chain cleavage activity and improve sugar chain transfer activity, to prepare antibodies in which the sugar chain Y is attached to the other heavy chain of the two heavy chains on the left and the right and in which the sugar chains attached to the two heavy chains on the left and the right are sugar chains structurally different from each other, followed by purification and isolation of the antibodies.
[5] The method according to [4], wherein the antibodies are purified and isolated by use of affinity chromatography using a carrier with an Fcγ receptor immobilized thereon as a ligand.
[6] The method according to [4] or [5], wherein the ENGase is EndoS.
[7] The method according to any of [4] to [6], wherein the ENGase modified so as to suppress sugar chain cleavage activity and retain sugar chain transfer activity is selected from the group consisting of EndoS D233Q, Endo S2 D184M, Endo S2 D184Q, EndoS D233Q/Q303L, D233Q/A303L/E350Q, and Endo M N175Q.
[8] The method according to any of [4] to [7], wherein a molecular weight of the sugar chain X to be attached in the step (ii) is larger than a molecular weight of the sugar chain Y to be attached in the step (iii).
[9] The method according to any of [4] to [8], wherein the produced population of antibodies comprises 90% or more of the antibodies in which the N-linked complex sugar chains attached to asparagine (Asn) at position 297 of CH domains of Fc regions of two heavy chains on the left and the right of each antibody are different from each other.
[10] The method according to any of [4] to [9], wherein N-acetylglucosamine (GlcNAc) at reducing ends of the N-linked complex sugar chains attached to asparagine (Asn) at position 297 located in the CH domains of the antibody Fc regions is fucosylated in the produced population of antibodies.
[11] A method for fractionating antibodies in a population of antibodies into a non-glycosylated antibody, an antibody glycosylated at one heavy chain, and an antibody glycosylated at two heavy chains by use of affinity chromatography using a carrier with an Fcγ receptor immobilized thereon as a ligand.
[12] The method according to [11], wherein the population of antibodies is treated with endo-β-N-acetylglucosaminidase (ENGase), and then, the antibodies in the population of antibodies are fractionated into a non-glycosylated antibody, an antibody glycosylated at one heavy chain, and an antibody glycosylated at two heavy chains by use of affinity chromatography using a carrier with an Fcγ receptor immobilized thereon as a ligand.

The present specification encompasses the contents disclosed in Japanese Patent Application No. 2020-002968 on which the priority of the present application is based.

### Advantageous Effects of Invention

A population of antibodies comprising homogeneous antibodies each having left-right asymmetric sugar chains can be prepared by cleaving structurally heterogeneous sugar chains with ENGase, followed by glycosylation using synthesized or isolated sugar-oxazoline and modified ENGase.

### Brief Description of Drawings

[Figure 1] Figure 1 is a diagram showing results of analyzing commercially available Herceptin with Tosoh TSKgel(R) FcR-IIIA-NPR.
[Figure 2] Figure 2 is a diagram showing the sugar chain structures of commercially available Herceptin. In the diagram, "Heterogeneous moiety" means that commercially available Herceptin includes antibody molecules differing in the structure of this moiety.
[Figure 3] Figure 3 is a diagram showing results of analyzing a deglycosylated antibody: Herceptin[GlcNAc-Fucose/GlcNAc-Fucose] with Tosoh TSKgel(R) FcR-IIIA-NPR.
[Figure 4] Figure 4 is a diagram showing the deglycosylated antibody: Herceptin[GlcNAc-Fucose/GlcNAc-Fucose].
[Figure 5] Figure 5 is a diagram showing results of purifying a deglycosylation reaction solution with Protein A.
[Figure 6] Figure 6 is a diagram showing the structure of SG-Oxazoline for use in glycosyltransfer reaction.
[Figure 7] Figure 7 is a diagram showing results of purifying a sugar chain transfer reaction solution containing SG-Ox with Protein A.
[Figure 8] Figure 8 is a diagram showing results of analyzing a glycosyltransfer reaction solution containing SG-Ox with Tosoh TSKgel(R) FcR-IIIA-NPR.
[Figure 9] Figure 9 is a diagram showing an antibody of Fr.1: Herceptin[GlcNAc-Fucose/GlcNAc-Fucose] .
[Figure 10] Figure 10 is a diagram showing results of analyzing the intact mass of Fr.1; Herceptin[GlcNAc-Fucose/GlcNAc-Fucose].
[Figure 11] Figure 11 is a diagram showing an antibody of Fr.2: Herceptin[SG-F/GlcNAc-Fucose].
[Figure 12] Figure 12 is a diagram showing results of analyzing the intact mass of Fr.2; Herceptin[SG-F/GlcNAc-Fucose].
[Figure 13] Figure 13 is a diagram showing an antibody ofFr.3: Herceptin[SG-F/SG-F].
[Figure 14] Figure 14 is a diagram showing results of analyzing the intact mass of the antibody of Fr.3: Herceptin[SG-F/SG-F].
[Figure 15] Figure 15 is a diagram showing the structure of G2-Oxazoline for use in glycosyltransfer reaction.
[Figure 16] Figure 16 is a diagram showing results of analyzing a glycosyltransfer reaction solution containing G2-Ox with Tosoh TSKgel(R) FcR-IIIA-NPR.
[Figure 17] Figure 17 is a diagram showing the structure of an antibody obtained by fractionation: Herceptin[G2-F/GlcNAc-Fucose].
[Figure 18] Figure 18 is a diagram showing results of analyzing the intact mass of the antibody obtained by fractionation: Herceptin[G2-F/GlcNAc-Fucose].
[Figure 19] Figure 19 is a diagram showing the structure of GO-Oxazoline for use in glycosyltransfer reaction.
[Figure 20] Figure 20 is a diagram showing results of analyzing a glycosyltransfer reaction solution containing GO-Ox with Tosoh TSKgel(R) FcR-IIIA-NPR.
[Figure 21] Figure 21 is a diagram showing the structure of an antibody obtained by fractionation: Herceptin[G0-F/GlcNAc-Fucose].
[Figure 22] Figure 22 is a diagram showing results of analyzing the intact mass of the antibody obtained by fractionation: Herceptin[G0-F/GlcNAc-Fucose].
[Figure 23] Figure 23 is a diagram showing the structure of SG-Oxazoline for use in glycosyltransfer reaction.
[Figure 24] Figure 24 is a diagram showing results of analyzing a glycosyltransfer reaction solution using M3-Ox with Tosoh TSKgel(R) FcR-IIIA-NPR.
[Figure 25] Figure 25 is a diagram showing the structure of an antibody obtained by fractionation: Herceptin[M3-F/GlcNAc-Fucose].
[Figure 26] Figure 26 is a diagram showing results of analyzing the intact mass of the antibody obtained by fractionation: Herceptin[M3-F/GlcNAc-Fucose].
[Figure 27] Figure 27 is a diagram showing results of purifying a sugar chain transfer reaction solution using G2-Ox for a single-SG1 chain antibody with Protein A.
[Figure 28] Figure 28 is a diagram showing results of analyzing a G2 transfer reaction solution for the single-SG1 chain antibody with Tosoh TSKgel(R) FcR-IIIA-NPR.
[Figure 29] Figure 29 is a diagram showing the structure of an antibody obtained by fractionation: Herceptin[SG-F/G2-F].
[Figure 30] Figure 30 is a diagram showing results of analyzing the intact mass of the antibody obtained by fractionation: Herceptin[SG-F/G2-F].
[Figure 31] Figure 31 is a diagram showing results of analyzing a SG transfer reaction solution for the single-GO chain antibody with Tosoh TSKgel(R) FcR-IIIA-NPR.
[Figure 32] Figure 32 is a diagram showing the structure of an antibody obtained by fractionation: Herceptin[SG-F/G0-F].
[Figure 33] Figure 33 is a diagram showing results of analyzing the intact mass of the antibody obtained by fractionation: Herceptin[SG-F/G0-F].
[Figure 34] Figure 34 is a diagram showing results of analyzing a G2 transfer reaction solution for the single-M3 chain antibody with Tosoh TSKgel(R) FcR-IIIA-NPR.
[Figure 35] Figure 35 is a diagram showing the structure of an antibody obtained by fractionation: Herceptin[G2-F/M3-F].
[Figure 36] Figure 36 is a diagram showing results of analyzing the intact mass of the antibody obtained by fractionation: Herceptin[G2-F/M3-F].

### Description of Embodiments

Hereinafter, the present invention will be described in detail.

Monoclonal antibodies prepared in CHO (Chinese hamster ovary) cells are currently used as antibody drugs. Protein structures consisting of heavy chains and light chains of the monoclonal antibodies thus prepared are homogeneous, whereas the structures of sugar chains are heterogeneous among the antibodies. In this context, the sugar chains attached to an antibody refer to N-linked complex sugar chains attached to asparagine (Asn) at position 297 located in the CH domains of Fc regions of the antibody. In the natural world, there also exist antibodies in which sugar chains attached to two heavy chains of each antibody are structurally different from each other, i.e., antibodies each having left-right asymmetric structures of sugar chains. However, in view of a population of antibodies, there also exist antibodies in which sugar chains attached to two heavy chains of each antibody have the same structure with each other, i.e., antibodies each having left-right symmetric sugar chains. The antibodies each having left-right asymmetric structures of sugar chains differ in the types of the sugar chains among the antibodies. Thus, no population of antibodies comprising homogeneous antibodies in which sugar chains attached to two heavy chains of each antibody are structurally different from each other has existed. On the other hand, a population of antibodies consisting of antibodies having left-right symmetric and homogeneous sugar chains has been able to be obtained by replacing the sugar chains of antibodies in a population of antibodies comprising antibodies having heterogeneous sugar chains with other sugar chains using endo-β-N-acetylglucosaminidase and a modified form thereof.

The present invention provides a method for preparing a population of antibodies comprising homogeneous antibodies each having left-right asymmetric sugar chains, which is impossible to attain by a conventional method. The present invention also provides a population of antibodies comprising homogeneous antibodies each having left-right asymmetric sugar chains, and the population is an isolated population. The population of antibodies refers to an antibody group including many antibody molecules. The population of antibodies is a population comprising a given proportion of the antibodies each having left-right asymmetric sugar chains, or a population comprising 100% of the antibodies each having left-right asymmetric sugar chains, i.e., a population consisting of the antibodies each having left-right asymmetric sugar chains. The antibodies each having left-right asymmetric sugar chains refer to antibodies in which sugar chains attached to asparagine (Asn) at position 297 of CH domains of Fc regions of two heavy chains on the left and the right of each antibody are sugar chains structurally different from each other. Alternatively, the antibodies refer to antibodies in which different sugar chains are attached to two monomer moieties of each antibody which is a dimer.

The population of antibodies comprising homogeneous antibodies each having left-right asymmetric sugar chains refer to a population of antibodies in which the types of sugar chains attached to CH domains of two heavy chains on the left and the right of each antibody molecule are homogeneous among the antibodies. Specifically, the population of antibodies comprising homogeneous antibodies each having left-right asymmetric sugar chains refer to a population of antibodies in which different sugar chains are attached to two heavy chains in each antibody, whereas the types of the sugar chains are the same among the antibodies and the sugar chains attached to each antibody are homogeneous among the antibodies.

In this context, the term "homogeneous" means that the population of antibodies comprises 70% or more, 75% or more, 80% or more, 85% or more, 90% or more, or 95% or more, preferably 98% or more, more preferably 99% or more, particularly preferably 100%, of antibody molecules having the same asymmetric sugar chains attached to heavy chains on the left and the right. The population of antibodies can also be referred to as an antibody composition. The antibody composition described herein may contain water, a buffer solution component, a stabilizing component, and the like. The population of antibodies may be an antibody solution in a liquid state or may be a freeze-dried product or a frozen product. The population of antibodies comprising homogeneous antibodies each having left-right asymmetric sugar chains has been isolated by chromatography and may therefore be referred to as an antibody fraction comprising homogeneous antibodies each having left-right asymmetric sugar chains.

In the method of the present invention, the sugar chains in the population of antibodies can be obtained by replacing the sugar chains of two heavy chains on the left and the right with sugar chains different from each other using modified endo-β-N-acetylglucosaminidase (ENGase).

Specifically, the sugar chains are replaced by, for example, a method given below. In the example given below, the sugar chains of two heavy chains on the left and the right of each antibody are respectively replaced with sugar chain X and sugar chain Y which is a sugar chain different from the sugar chain X.

In the following method, the antibodies themselves can be purified using Protein A or the like.

### (1) Cleavage of original sugar chains attached to antibody

The original sugar chains attached to each antibody are cleaved using endo-β-N-acetylglucosaminidase (ENGase). In this respect, both the sugar chains attached to two heavy chains on the left and the right of each antibody are cleaved. The ENGase is an enzyme that hydrolyzes N,N'-diacetylchitobiose present on the reducing end side of N-linked sugar chains of a glycoprotein such as an antibody to liberate sugar chains in an endo form. The ENGase cleaves the sugar chains such that one N-acetylglucosamine (GlcNAc) residue remains at the reducing ends of the N-linked complex sugar chains attached to asparagine (Asn) at position 297 located in the CH domains of the antibody Fc regions. The N-acetylglucosamine at the reducing ends may or may not be fucosylated. The cleavage of a sugar chain is called deglycosylation. This consequently produces antibodies to which only GlcNAc is attached without sugar chains attached thereto.

This enzymatic reaction can be performed for 5 to 30 hours, preferably 10 to 25 hours, by mixing the antibodies with the ENGase at pH 6 to 9, preferably pH 6 to 8, at 15 to 40°C, preferably 25 to 35°C. The amounts of the antibodies and the ENGase added can be appropriately set. For example, 5 to 100 µL of 0.2 to 20 mg/mL ENGase can be added to 0.1 to 10 mL of a 5 to 100 mg/mL antibody solution.

The endo-P-N-acetylglucosaminidase (ENGase) is preferably EndoS derived from *Streptococcus pyogenes.* The EndoS is described in Collin, M., Olsen, A. (2001), EndoS, a novel secreted protein from Streptococcus pyogenes with endoglycosidase activity on human IgG. EMBO J. 20, 3046-3055, and its amino acid sequence is also disclosed therein.

Then, the antibodies from which the sugar chains have been cleaved can be purified and isolated. The purification can be performed by use of chromatography. In this respect, the purification can be performed by affinity chromatography using a carrier, such as a column, with an Fcγ receptor FcγRIIIa immobilized thereon as a ligand. The Fcγ receptor is a receptor protein against an immunoglobulin Fc site and is a receptor protein capable of recognizing structural change in Fc region caused by a N-linked sugar chain of an antibody. Examples of the Fcγ receptor include FcγRIIIa. In the affinity chromatography using a column with an Fcγ receptor immobilized thereon, an antibody having sugar chain structures free of fucose, or an antibody having terminal galactose in sugar chain structures has larger binding affinity and a delayed elution time.

Examples of the column with FcγRIIIa immobilized thereon include TSKgel(R) FcR-IIIA-NPR (Tosoh Corp.).

This step is the step of cleaving sugar chains attached to two heavy chains on the left and the right of each antibody in an antibody composition with endo-P-N-acetylglucosaminidase (ENGase), and purifying and isolating antibodies from which the sugar chains of both the heavy chains have been cleaved.

### (2) Preparation of antibody having sugar chain on only one heavy chain

Subsequently, the antibodies from which both the sugar chains attached to the two heavy chains have been cleaved are mixed with sugar chain X in an oxazoline form or an oxazoline-generating sugar derivative, the sugar chain of which is sugar chain X, and this sugar chain is attached thereto using ENGase modified so as to suppress sugar chain cleavage activity and improve sugar chain transfer activity, i.e., ENGase having glycosyltransfer ability while its hydrolysis ability is suppressed. Examples of the oxazoline-generating sugar derivative include glycopeptides and p-nitrophenyl oligosaccharides. The ENGase modified so as to suppress sugar chain cleavage activity and improve sugar chain transfer activity includes all ENGases that retain glycosyltransfer ability while its sugar chain cleavage ability is suppressed. Examples thereof include EndoS having a D233Q mutation (substitution of aspartic acid (D) at position 233 by glutamine (Q)). This mutant antibody is referred to as EndoS D233Q. The ENGase modified so as to suppress sugar chain cleavage activity and improve sugar chain transfer activity also includes Endo S2 D184M and Endo S2 D184Q (Li, T. et al., J. Biol. Chem. 291, 16508-16518,(2016)), and Endo M N175Q (Katoh, T. et al., The Journal of Biological Chemistry, 291, 23305-23317). The enzyme further includes EndoS D233Q/Q303L and D233Q/A303L/E350Q (International Publication No. WO2017/010559).

The sugar chain in an oxazoline form is obtained by preparing a sugar chain having GlcNAc at the reducing end into an oxazoline form using an oxazoline-forming agent. The sugar chain in an oxazoline form thus prepared is referred to as a sugar chain in an oxazoline form or sugar chain-oxazoline. The sugar chain X in an oxazoline form is used as a substrate for sugar chain transfer to GlcNAc-antibodies with the modified ENGase of the present invention to attach the sugar chain X to the antibodies. Specifically, an oxazoline sugar chain (azide oxazoline sugar chain) serving as a donor is attached to the N-acetylglucosamine (GlcNAc) residues of the acceptor antibodies from which the sugar chains have been cleaved. In this reaction, the oxazoline ring of the donor reacts with position 4 of the antibody N-acetylglucosamine (GlcNAc) residues to form a chitobiose structure for attachment. Examples of the oxazoline-forming agent include CDMBI (2-chloro-1,3-dimethyl-1H-benzimidazol-3-ium chloride, Fushimi Pharmaceutical Co., Ltd.) and DMC (2-chloro-1,3-dimethyl-imidazolinium chloride). The oxazoline form can be obtained by a method described in J. Org. Chem. 74, 5, 2210 (2009). Also, the sugar chain in an oxazoline form is available from Fushimi Pharmaceutical Co., Ltd.

A sugar derivative that generates oxazoline upon cleavage of its sugar chain with the modified ENGase may be used. A sugar chain in an oxazoline form is generated from the sugar derivative, and the sugar chain in an oxazoline form serving as a donor is attached *in situ* to the N-acetylglucosamine (GlcNAc) residues of the acceptor antibodies from which the sugar chains have been cleaved. A method using the oxazoline-generating sugar derivative is described in Manabe, S. et al., R. Soc. Open Sci. 5, 171521 (2018) and Iwamoto, M. et al., PLoS One 13: e0193534 (2018).

Both in the case of using the sugar chain in an oxazoline form and in the case of using the oxazoline-generating sugar derivative, it can be said that "sugar is transferred by using oxazoline as an intermediate" because the sugar chain in an oxazoline form is used as an intermediate.

This enzymatic reaction can be performed for 5 to 30 hours, preferably 10 to 25 hours, by mixing the antibodies from which both the sugar chains on the left and the right have been cleaved with the sugar chain in an oxazoline form or the oxazoline-generating sugar derivative, the sugar chain of which is sugar chain X, and the modified ENGase at pH 6 to 9, preferably pH 6 to 8, at 15 to 40°C, preferably 25 to 35°C. The amounts of the antibodies and the ENGase added can be appropriately set. For example, 0.1 to 100 µL of 0.2 to 20 mg/mL modified ENGase can be added to 0.1 to 10 mL of a 5 to 100 mg/mL solution of the antibodies from which both the sugar chains on the left and the right have been cleaved.

In this respect, antibodies each having the sugar chain X on only one of the two heavy chains on the left and the right can be obtained as principal products by adjusting the amount of the sugar chain X in an oxazoline form or the oxazoline-generating sugar derivative, the sugar chain of which is sugar chain X. For example, the sugar chain X in an oxazoline form or the oxazoline-generating sugar derivative, the sugar chain of which is sugar chain X can be added at 25 µM to 5.0 mM.

This reaction also produces a non-glycosylated antibody in which no sugar chain is attached to the two heavy chains on the left and the right, and a double-sugar chain antibody in which the sugar chain X is attached to the two heavy chains on the left and the right. The elution time is shorter in the order of a non-glycosylated antibody, an antibody glycosylated at one heavy chain, and an antibody glycosylated at two heavy chains. In order to prepare the population of antibodies having homogeneous structures of left-right asymmetric sugar chains according to the present invention, only a single-sugar chain antibody having the sugar chain X on only one of the two heavy chains on the left and the right needs to be purified and isolated. In order to purify the single-sugar chain antibody, the purification can be performed by affinity chromatography using the column with an Fcγ receptor FcγRIIIa immobilized thereon.

This step is the step of mixing a population of the antibodies from which the sugar chains of both the heavy chains have been cleaved, obtained in the step (1), any sugar chain X in an oxazoline form or an oxazoline-generating sugar derivative, the sugar chain of which is sugar chain X, and ENGase modified so as to suppress sugar chain cleavage activity and improve sugar chain transfer activity, to prepare antibodies in which the sugar chain X is attached to only one of the two heavy chains on the left and the right, followed by purification and isolation of the antibodies.

### (3) Preparation of antibody having left-right asymmetric sugar chains

After the single-sugar chain antibody having the sugar chain X on only one of the two heavy chains on the left and the right is purified by the method of (2), sugar chain Y is attached to the other heavy chain without a sugar chain attached thereto. The sugar chain Y can be attached in the same manner as in the sugar chain X. Specifically, the enzymatic reaction can be performed for 5 to 30 hours, preferably 10 to 25 hours, by mixing the antibodies with the modified ENGase at pH 6 to 9, preferably pH 6 to 8, at 15 to 40°C, preferably 25 to 35°C. The amounts of the antibodies and the ENGase added can be appropriately set. For example, 5 to 100 µL of 0.2 to 20 mg/mL ENGase can be added to 0.1 to 10 mL of a 5 to 100 mg/mL antibody solution.

As a result, antibodies each having left-right asymmetric sugar chains in which different types of sugar chains are attached to the two heavy chains on the left and the right can be prepared. The antibodies each having left-right asymmetric sugar chains in which different types of sugar chains are attached to the two heavy chains on the left and the right can be purified by use of affinity chromatography using a column with FcγRIIIa immobilized thereon to isolate and produce a population of antibodies comprising homogeneous antibodies each having left-right asymmetric sugar chains in which different types of sugar chains are attached to the two heavy chains on the left and the right.

This step is the step of mixing the antibodies in which the sugar chain X is attached to only one of the two heavy chains on the left and the right, obtained in the step (2), sugar chain Y in an oxazoline form which is structurally different from the sugar chain X, or an oxazoline-generating sugar derivative, the sugar chain of which is sugar chain Y, and ENGase modified so as to suppress sugar chain cleavage activity and improve sugar chain transfer activity, to prepare antibodies in which the sugar chain Y is attached to the other heavy chain of the two heavy chains on the left and the right and in which the sugar chains attached to the two heavy chains on the left and the right are sugar chains structurally different from each other, followed by purification and isolation of the antibodies.

The sugar chains to be attached to the antibodies are appropriately selected according to a purpose. Examples thereof include high-mannose sugar chains having a structure where an oligomer of mannose (Man) is bonded to diacetylchitobiose (GlcNAc-GlcNAc) including N-acetylglucosamine (GlcNAc) at reducing ends, complex sugar chains in which Man and at least one of GlcNAc, galactose (Gal), and sialic acid (Neu5Ac) are bonded to diacetylchitobiose, and hybrid sugar chains having a sugar chain structure with a hybrid of high-mannose and complex forms with diacetylchitobiose. Examples of the high-mannose sugar chain include sugar chains called Man3, Man5, Man6, Man8 and Man9 having 3, 5, 6, 8 and 9 bonded mannoses, respectively. Examples of the complex sugar chain include sugar chains called triantennary form, tetraantennary form, asialo biantennary form, agalacto biantennary form, bisecting biantennary form, and sialo biantennary form. There are various structures of sugar chains depending on the presence or absence of sialic acid, the presence or absence of core fucose, the presence or absence of a branch, etc. Each sugar chain is represented by an abbreviation such as SG or M3. A sugar chain having sialic acid (sialyl sugar chain) is represented by SG; a high-mannose sugar chain is represented by M3, M5, or the like; and a sugar chain free of galactose is represented by G0; a sugar chain containing one galactose is represented by G1; and a sugar chain containing two galactoses is represented by G2. A sugar chain further having fucose is represented by M3-F, GO-F, or G2-F. A sugar chain further having bonded bisecting N-acetylglucosamine (indicated by B) is represented by G0B, G0B, or the like. The whole sugar chain structure of an antibody (IgG) molecule can be represented by, for example, [SG-F/SG-F], [SG-F/G2-F], [SG-F/G0-F], or [G2-F/M3-F], using sugar chains attached to heavy chains on the left and the right. In these examples, [SG-F/SG-F] is an antibody having left-right symmetric sugar chains, and [SG-F/G2-F], [SG-F/G0-F], or [G2-F/M3-F] is an antibody having left-right asymmetric sugar chains. The name of an antibody is described before these terms so as to indicate, for example, Herceptin[SG-F/G2-F]. This example is Herceptin having left-right asymmetric sugar chains in which SG-F is attached to one heavy chain and G2-F is attached to the other heavy chain.

An antibody from which both the sugar chains of two heavy chains on the left and the right have been cleaved is represented by [GlcNAc/GlcNAc], [GlcNAc-Fucose/GlcNAc-Fucose], or the like. The former one represents an antibody from which the sugar chains of both the heavy chains on the left and the right have been removed such that a N-acetylglucosamine (GlcNAc)1 residue remains. The latter one represents an antibody in which the remaining GlcNAc is fucosylated. An antibody in a state where a sugar chain is attached to only one of the two heavy chains on the left and the right is represented by [SG-F/GlcNAc-Fucose] or the like. This antibody represents an antibody in which SG-F is attached to only one of the heavy chains and fucosylated GlcNAc remains on the other heavy chain without a sugar chain attached thereto.

Each sugar chain may contain an azide group, an alkyne group, or the like. The sugar chain having such a group can be used in the synthesis of an antibody-drug conjugate (ADC).

A chemically synthesized non-natural sugar chain may be introduced to the antibodies. Examples of the non-natural sugar chain include non-human sugar chains containing N-glycolylneuraminic acid (NeuGc) instead of N-acetylneuraminic acid, or galactose such as α1-3 galactose (Galα1-3Gal) at the non-reducing ends. These sugar chains have immunogenicity and need to be removed from antibody drug compositions. However, antibodies having these sugar chains can be synthesized as standards by the method of the present invention.

The molecular weight of the sugar chain X to be attached firstly in (2) is desirably smaller than that of the sugar chain Y to be attached secondly. This is because, if a sugar chain having a smaller molecular weight is attached firstly, the sugar chain attached firstly is easily cleaved by hydrolysis upon attachment of a sugar chain to be attached secondly.

The present invention also encompasses a method for fractionating antibodies in a population of antibodies into a non-glycosylated antibody, an antibody glycosylated at one heavy chain, and an antibody glycosylated at two heavy chains by use of affinity chromatography using a carrier with an Fcγ receptor immobilized thereon as a ligand. The method can be performed, for example, after partial cleavage of sugar chains by the treatment of the population of antibodies with endo-β-N-acetylglucosaminidase (ENGase).

The antibodies which are prepared by the method of the present invention may be antibodies having core fucose in which GlcNAc at the base of each N-linked sugar chain is fucosylated, or may be antibodies having no core fucose. Their origin may be a human or may be a non-human animal including a rodent such as a mouse or a rat. The class of the antibodies may be any of IgG, IgM, IgA, IgD, and IgE.

The method of the present invention can produce antibody drugs, antibody-drug conjugates (ADCs), or antibodies for use as antibody standards in the form of antibodies having homogeneous structures of left-right asymmetric sugar chains. Biosimilars or biobetters can be produced by modifying the sugar chains of antibody drugs. Moreover, antibody standards differing in sugar chain structure can be obtained.

Examples of the antibody drug include, but are not limited to, the following antibodies:
trastuzumab (Herceptin(R)), rituximab (Rituxan(R)), mogamulizumab (Poteligeo(R)), adalimumab, alirocumab, alemtuzumab, ixekizumab, idarucizumab, ipilimumab, infliximab, ustekinumab, eculizumab, evolocumab, elotuzumab, ofatumumab, omalizumab, canakinumab, gemtuzumab ozogamicin, golimumab, secukinumab, cetuximab, certolizumab pegol, denosumab, tocilizumab, trastuzumab, trastuzumab emtansine, natalizumab, nivolumab, basiliximab, panitumumab, palivizumab, brentuximab vedotin, brodalumab, bevacizumab, pembrolizumab, pertuzumab, mepolizumab, ranibizumab, and ramucirumab.

### Examples

The present invention will be specifically described with reference to Examples given below. However, the present invention is not limited by these Examples.

### Preparation of antibody having left-right asymmetric sugar chains

The antibody used was an anti-Her2 antibody Herceptin(R) (trastuzumab). Trastuzumab is an antibody drug for breast cancer and stomach cancer. Sugar chain moieties were synthesized as follows.

### [Example 1] Preparation of antibody having only reducing end GlcNAc (fucosylated); Herceptin[GlcNAc-Fucose/GlcNAc-Fucose]

1-1. The commercially available product Herceptin (manufactured by Chugai Pharmaceutical Co., Ltd., Injection 150, 177 mg) was dissolved in 15 mL of a 100 mM phosphate buffer solution (pH 6.5). Then, the solution was concentrated to approximately 5 mL with Sartorius Vivaspin Turbo 15 (50 K) (3,000 × g, 4°C). To the concentrate, 10 mL of a 100 mM phosphate buffer solution (pH 6.5) was added, and concentration to 5 mL was repeated three times.

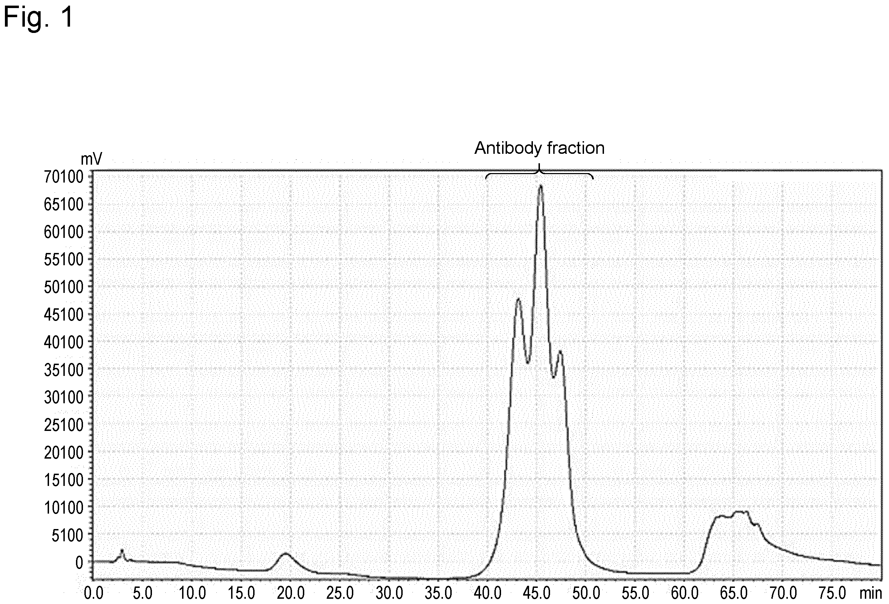

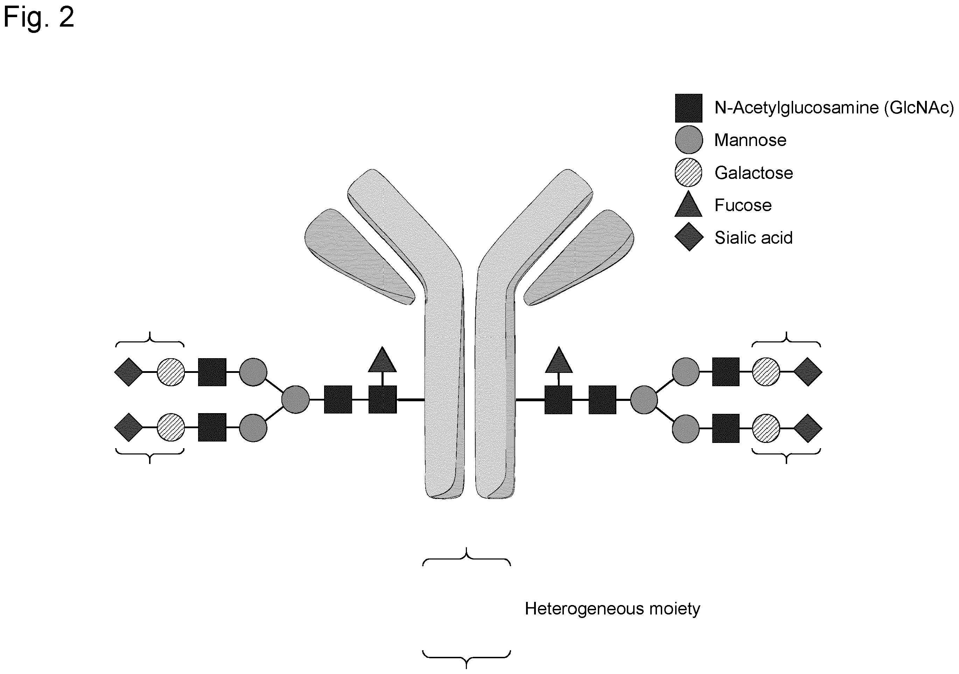

Figure 1 shows results of analyzing the commercially available product Herceptin with TSKgel(R) FcR-IIIA-NPR column from Tosoh Corp. (analysis conditions 1). An image diagram of the structure of this antibody is shown in Figure 2.

1-2. To the Herceptin solution, 10 µL of EndoS (2.0 mg/mL) was added, and the mixture was reacted at 30°C for 18 hours. The consumption of the starting materials thus reacted was confirmed by HPLC.

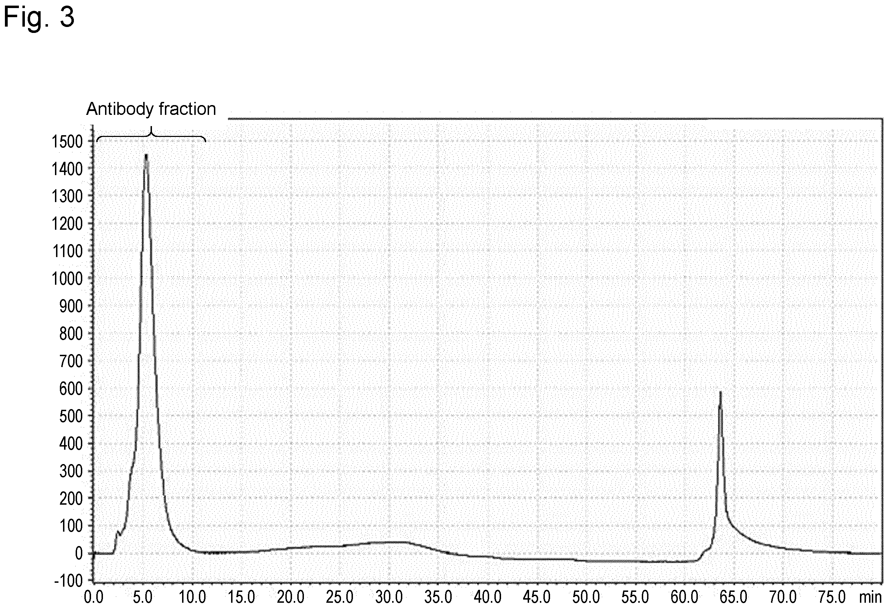

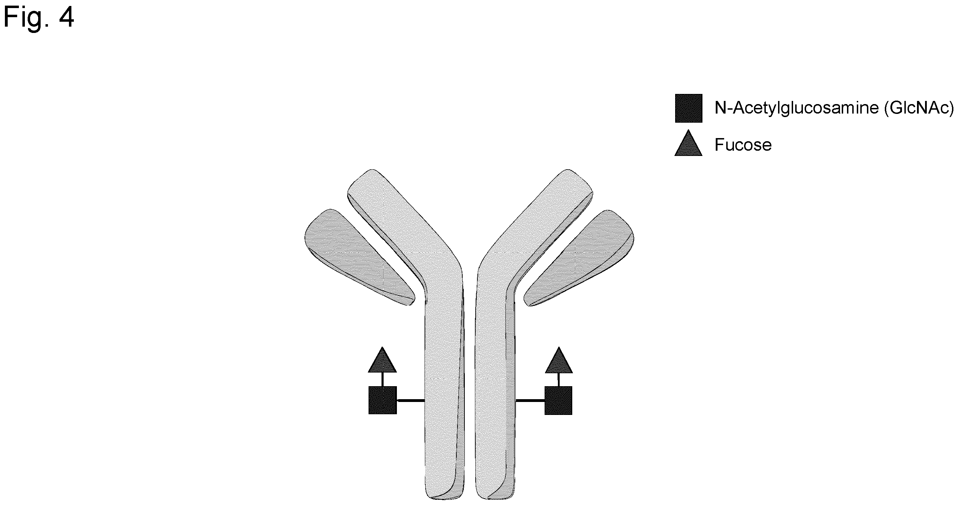

Analysis results are shown in Figure 3 (analysis conditions 1). An image diagram of the structure of the antibody thus deglycosylated is shown in Figure 4.

Analysis conditions 1
Apparatus: Shimadzu HPLC system Prominence
Column: Tosoh TSKgel(R) FcR-IIIA-NPR 4.6 mm I.D. × 10 cm, 5 m
Column temperature: 25°C
Flow rate: 1 ml/min
Detection: Ex. 280 nm, Em. 348 nm
Mobile phase A: 50 mM citrate buffer (pH 6.5)
Mobile phase B: 50 mM citrate buffer (pH 4.5)

### Gradient conditions

1. 0-2 min A 100%
2. 2-45 min A 100-0%
3. 45-55 min A 0%
4. 55-60 min A 0-100%
5. 60-80 min A 100%

1-3. Antibodies were purified from the reaction solution with a Protein A column. The reaction solution was fractionated 10 times.

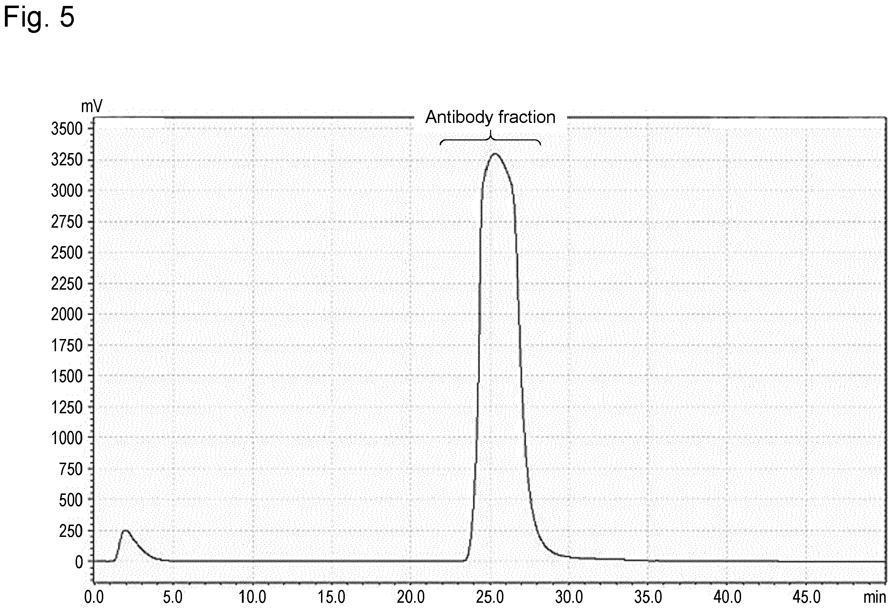

A chromatogram obtained by the antibody purification is shown in Figure 5. A peak around a retention time of 25 minutes was recovered as antibodies.

Purification conditions
Apparatus: Shimadzu HPLC system
Column: Tosoh ToyoScreen AF-Protein A HC-650F 1 ml
Column temperature: 25°C
Flow rate: 1 ml/min
Detection: UV 280 nm
Mobile phase A: 0.1 M phosphate buffer solution (pH 6.5)
Mobile phase B: 0.1 M citrate buffer (pH 3.5)

### Gradient conditions

1. 0-15 min A 100%
2. 15-15.01 min A 100-0%
3. 15.01-25 min A 0%
4. 25-25.01 min A 0-100%
5. 25.01-50 min A 100%

1-4. The recovered fractions were concentrated by ultrafiltration with Sartorius Vivaspin Turbo 15 (50 K). Then, the buffer was replaced with a 50 mM citrate buffer (pH 6.5) (2,460 × g, 4°C).

1-5. The protein concentration of the obtained Herceptin solution was measured using NanoDrop(TM) 2000C manufactured by Thermo Fisher Scientific Inc. (attached software, mode; Protein A280, Type; IgG for measurement) and was consequently 165 mg (30 mL, 5.5 mg/mL).

### [Example 2] Preparation of antibody having sugar chain at one location 2-1. Preparation of Herceptin[SG-F/GlcNAc-Fucose]

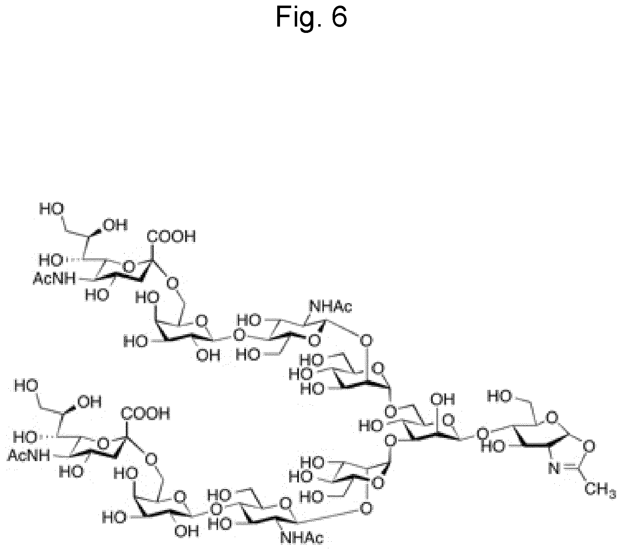

2-1-1. Into 995 µL of a 100 mM phosphate buffer solution (pH 6.5), Herceptin (20 mg; 2 mL) deglycosylated with ENGase, SG-Oxazoline (manufactured by Fushimi Pharmaceutical Co., Ltd.; 1 mM; 1000 µL, Figure 6; hereinafter, referred to as SG-Ox), and EndoS D233Q (4.35 µg; 1 µL) were added, and the mixture was reacted at 30°C for 12 hours.

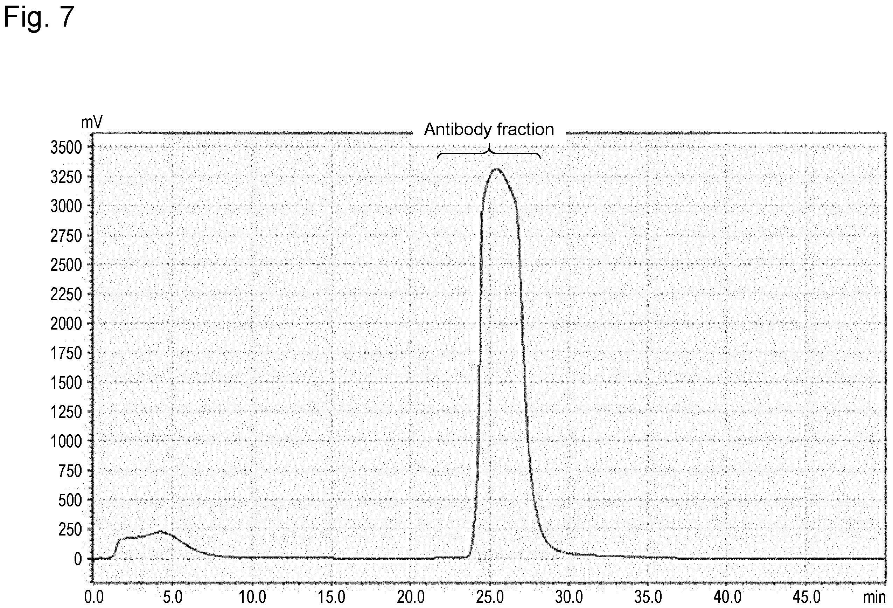

2-1-2. Antibodies were purified from the reaction solution with a Protein A column. A chromatogram obtained by the fractionation is shown in Figure 7. A peak around a retention time of 25 minutes (Antibody fraction of Figure 7) was recovered as antibodies.

Purification conditions
Apparatus: Shimadzu HPLC system
Column: Tosoh ToyoScreen AF-Protein A HC-650F 1 ml
Column temperature: 25°C
Flow rate: 1 ml/min
Detection: UV 280 nm
Mobile phase A: 0.1 M phosphate buffer solution (pH 6.5)
Mobile phase B: 0.1 M citrate buffer (pH 3.5)

### Gradient conditions

1. 0-15 min A 100%
2. 15-15.01 min A 100-0%
3. 15.01-25 min A 0%
4. 25-25.01 min A 0-100%
5. 25.01-50 min A 100%

2-1-3. The recovered fractions were concentrated by ultrafiltration with Sartorius Vivaspin Turbo 15 (50 K). Then, the buffer was replaced with a 50 mM citrate buffer (pH 6.5) (2,460 × g, 4°C).

2-1-4. The protein concentration of the obtained Herceptin solution was measured using NanoDrop(TM) 2000C manufactured by Thermo Fisher Scientific Inc. (attached software, mode; Protein A280, Type; IgG for measurement) and was consequently 19 mg (6 mL, 3.3 mg/mL).

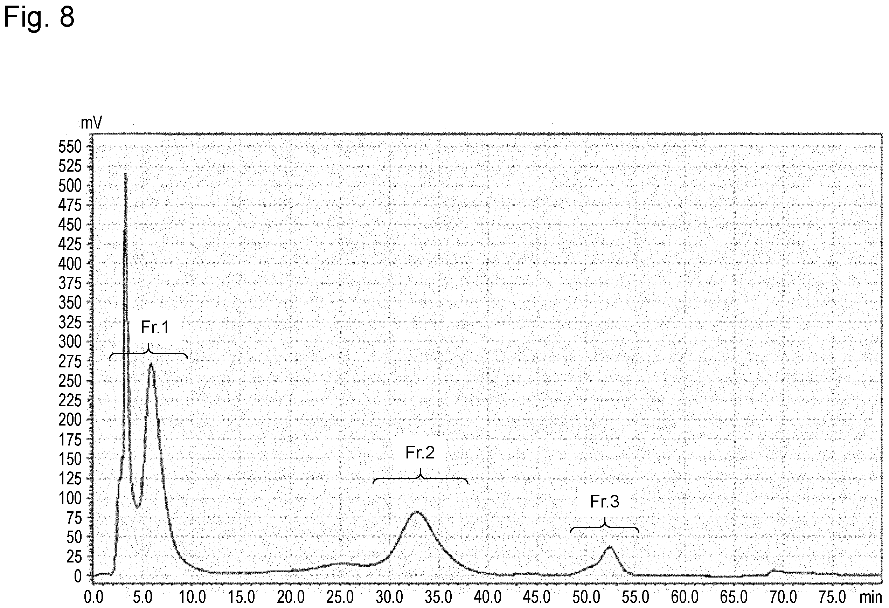

2-1-5. The Herceptin antibodies obtained by purification were fractionated using Tosoh TSKgel(R) FcR-IIIA-NPR. The fractionation results are shown in Figure 8. The peaks shown in Figure 8 (Fr.1, Fr.2 and Fr.3 of Figure 8) were obtained by the fractionation.

Fractionation conditions
Apparatus: Shimadzu HPLC system Prominence
Column: Tosoh TSKgel(R) FcR-IIIA-NPR 4.6 mm I.D. × 10 cm, 5 µm
Column temperature: 25°C
Flow rate: 1 ml/min
Detection: UV 280 nm
Mobile phase A: 50 mM citrate buffer (pH 6.5)
Mobile phase B: 50 mM citrate buffer (pH 4.5)

### Gradient conditions

1. 0-2 min A 100%
2. 2-45 min A100-0%
3. 45-55 min A 0%
4. 55-60 min A 0-100%
5. 60-80 min A 100%

2-1-6. The obtained fractions were designated as Fr.1 (2-10 min), Fr.2 (28-37 min), and Fr.3 (48-55 min) in the ascending order of the retention time and each subjected to intact mass analysis by LC-MS/MS.

Intact mass analysis
Apparatus: Waters Acquity H-Class Bio UHPLC System with Vion IMS Qtof
Column: Waters MassPREP Desalting Column
Column temperature: 80°C
Mobile phase: solution A: 0.1% formic acid
solution B: acetonitrile
Gradient conditions are shown in Table 1.

**[Table 1]**

| No. | Time (min) | Flow (ml/min) | A (%) | B (%) |
|---|---|---|---|---|
| 1 | 0 | 0.5 | 95 | 5 |
| 2 | 0.5 | 0.5 | 95 | 5 |
| 3 | 0.51 | 0.2 | 95 | 5 |
| 4 | 3 | 0.2 | 10 | 90 |
| 5 | 3.1 | 0.5 | 10 | 90 |
| 6 | 3.4 | 0.5 | 10 | 90 |
| 7 | 3.6 | 0.5 | 95 | 5 |
| 8 | 3.8 | 0.5 | 95 | 5 |

m/z range: 400-4000
Capillary voltage: 3.00 kV
Cone voltage: 150 V
Source temperature: 150°C
Desolvation temperature: 600°C
Deconvolution Software: Waters UNIFI software v1.8.2.

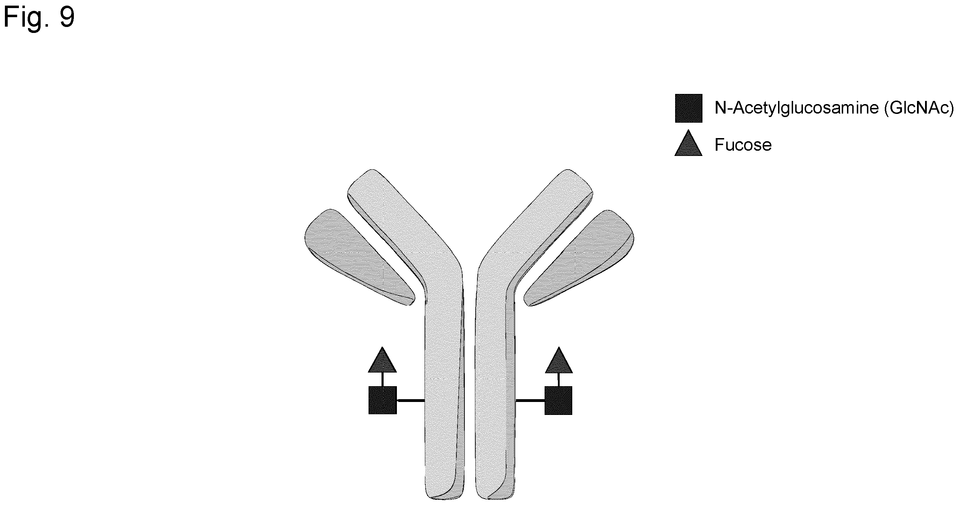

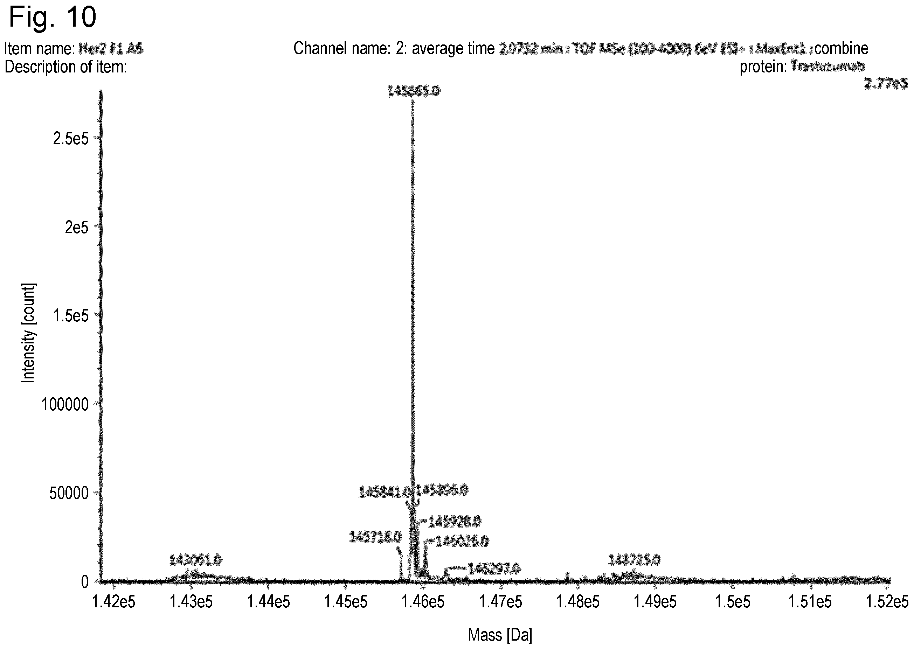

Fr.1 had a mass of 145865, revealing a structure (Figure 9) having one GlcNAc-Fucose pair attached to Asn297 (Figure 10).

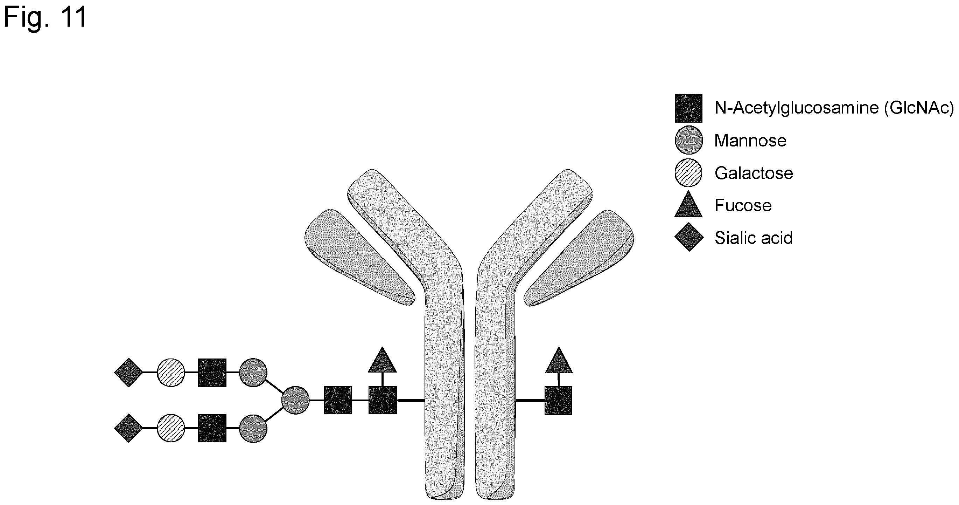

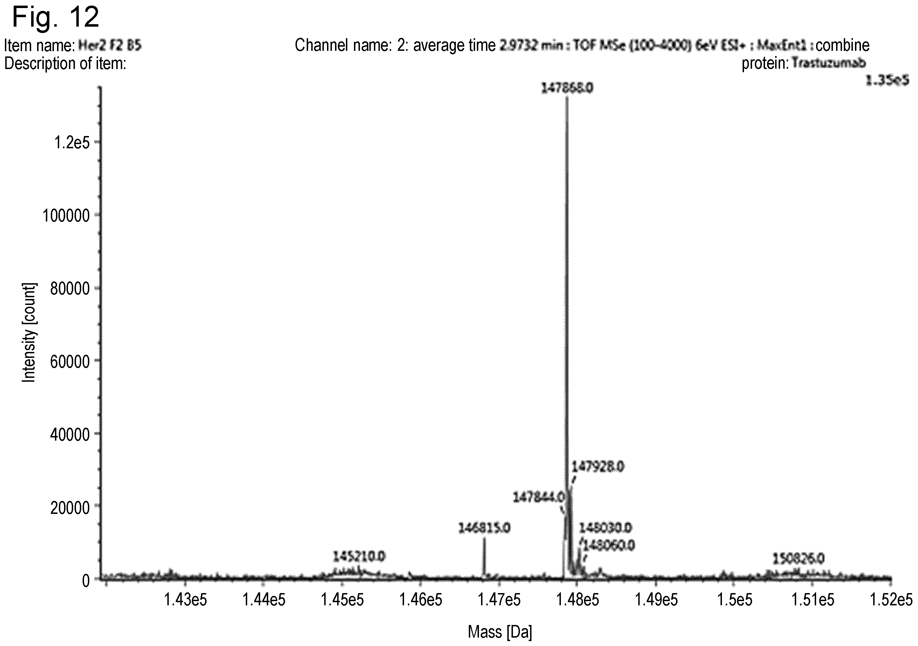

Fr.2 had a mass of 147868, revealing a structure (Figure 11) having SG attached to one GlcNAc-Fucose site of Asn297 (Figure 12).

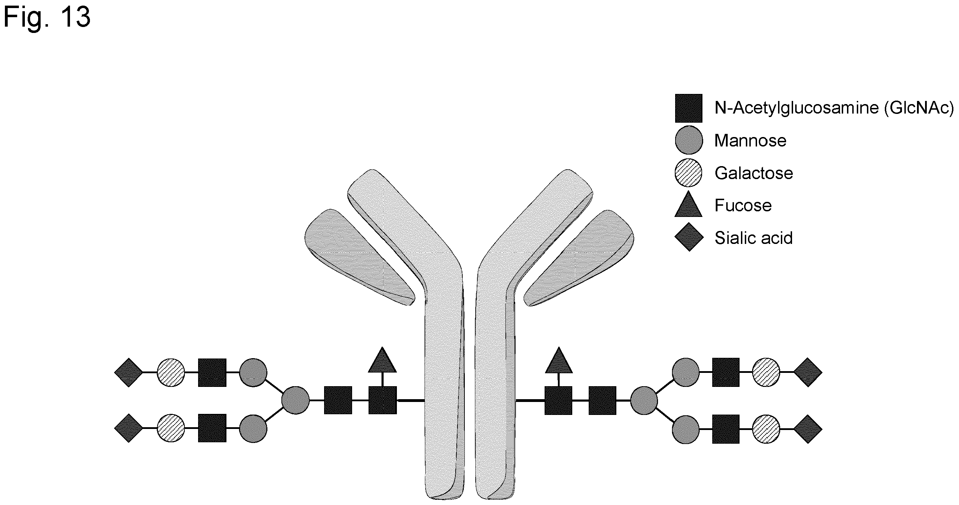

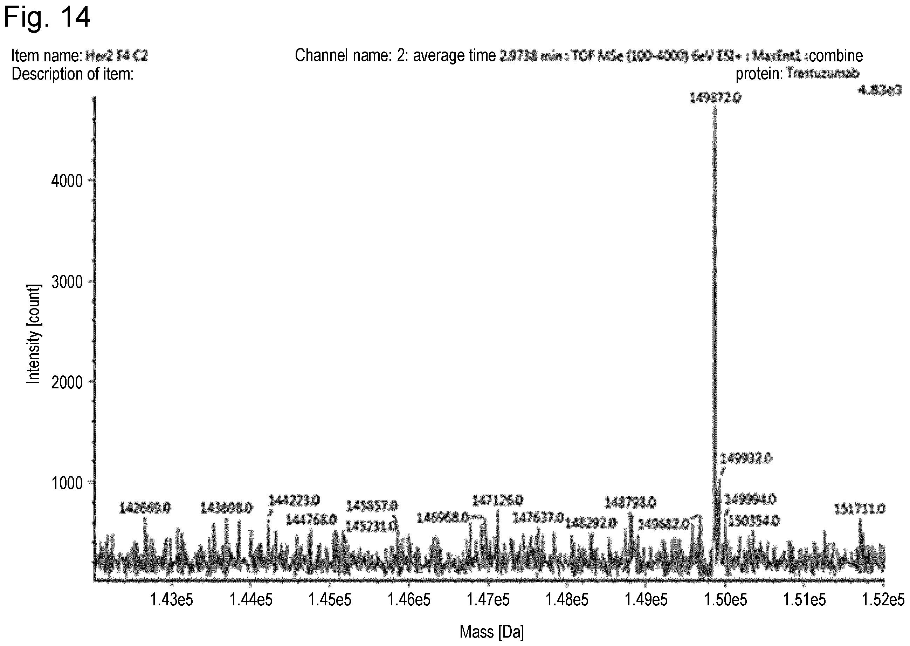

Fr.3 had a mass of 149872, revealing a structure (Figure 13) having SG attached to both of two GlcNAc-Fucose sites of Asn297 (Figure 14).

Thus, use of Tosoh TSKgel(R) FcR-IIIA-NPR enables products to be divided into a product having only GlcNAc-Fucose derived from sugar chains, a product having a sugar chain at only one location, and a product having sugar chains at two locations.

### 2-2. Preparation of Herceptin[G2-F/GlcNAc-Fucose]

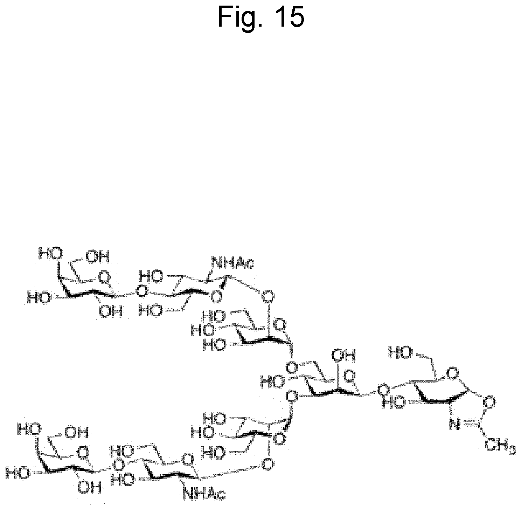

2-2-1. Into 98 µL of a 100 mM phosphate buffer solution (pH 6.5), Herceptin (2 mg; 200 µL) deglycosylated with ENGase, G2-Oxazoline (manufactured by Fushimi Pharmaceutical Co., Ltd.; 1 mM; 1000 µL, Figure 15; hereinafter, referred to as G2-Ox), and EndoS D233Q (4.35 µg; 2 µL) were added, and the mixture was reacted at 30°C for 12 hours. Antibodies in the solution thus reacted were purified by the same procedures as in 2-1-2.

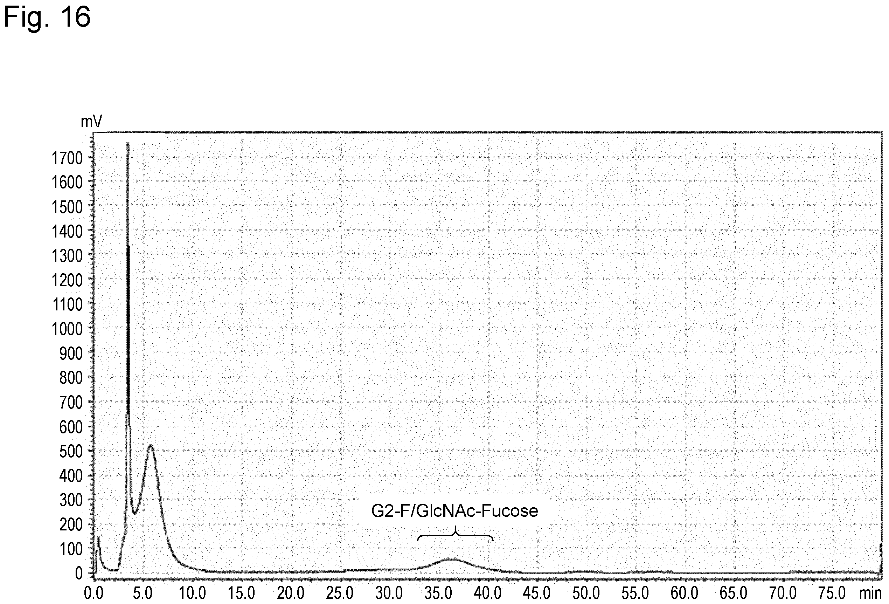

2-2-2. The Herceptin antibodies obtained by purification were fractionated using Tosoh TSKgel(R) FcR-IIIA-NPR. The fractionation results are shown in Figure 16. A peak around a retention time of 36 minutes (G2F/GlcNAc-Fucose of Figure 16) was obtained by the fractionation.

Fractionation conditions
Apparatus: Shimadzu HPLC system Prominence
Column: Tosoh TSKgel(R) FcR-IIIA-NPR 4.6 mm I.D. × 10 cm, 5 µm
Column temperature: 25°C
Flow rate: 1 ml/min
Detection: UV 280 nm
Mobile phase A: 50 mM citrate buffer (pH 6.5)
Mobile phase B: 50 mM citrate buffer (pH 4.5)

### Gradient conditions

1. 0-2min A 100%
2. 2-45min A100-0%
3. 45-55min A 0%
4. 55-60min A 0-100%
5. 60-80min A 100%

2-2-3. The peak obtained by fractionation was subjected to intact mass analysis by LC-MS/MS.
Intact mass analysis
Apparatus: Waters Acquity H-Class Bio UHPLC System with Vion IMS Qtof
Column: Waters MassPREP Desalting Column
Column temperature: 80°C
Mobile phase: solution A: 0.1% formic acid
solution B: acetonitrile
Gradient conditions are shown in Table 2.

**[Table 2]**

| No. | Time (min) | Flow (ml/min) | A (%) | B (%) |
|---|---|---|---|---|
| 1 | 0 | 0.5 | 95 | 5 |
| 2 | 0.5 | 0.5 | 95 | 5 |
| 3 | 0.51 | 0.2 | 95 | 5 |
| 4 | 3 | 0.2 | 10 | 90 |
| 5 | 3.1 | 0.5 | 10 | 90 |
| 6 | 3.4 | 0.5 | 10 | 90 |
| 7 | 3.6 | 0.5 | 95 | 5 |
| 8 | 3.8 | 0.5 | 95 | 5 |

m/z range: 400-4000
Capillary voltage: 3.00 kV
Cone voltage: 150 V
Source temperature: 150°C
Desolvation temperature: 600°C
Deconvolution Software: Waters UNIFI software v1.8.2.

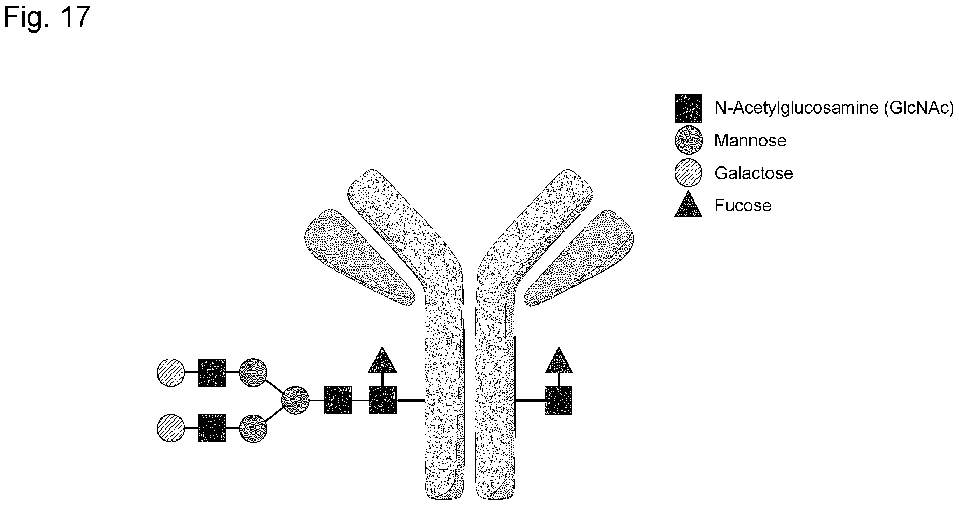

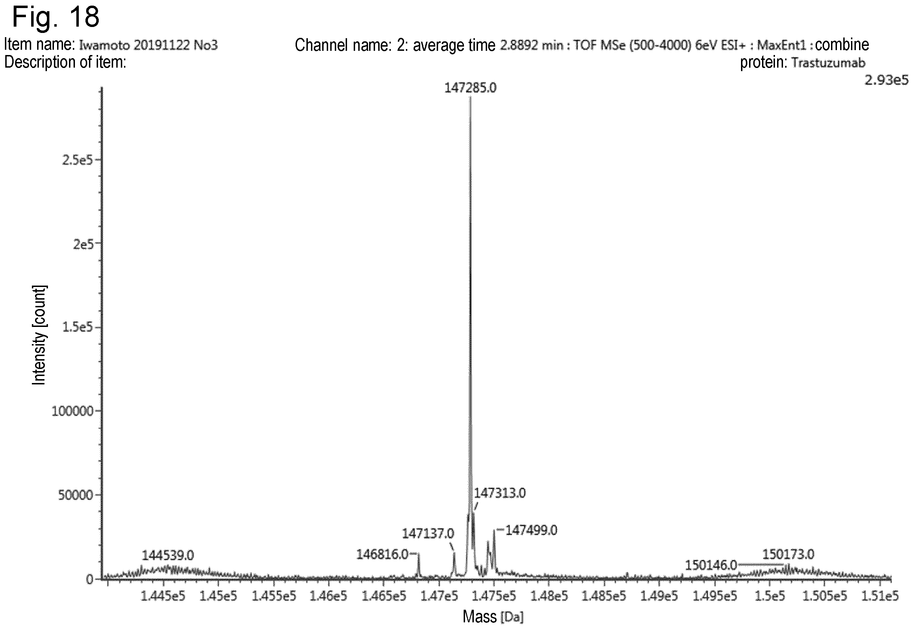

The detected mass was 147285, revealing a structure (Figure 17) having G2-F attached to one GlcNAc-Fucose site of Asn297 (Figure 18).

### 2-3. Preparation of Herceptin[G0-F/GlcNAc-Fucose]

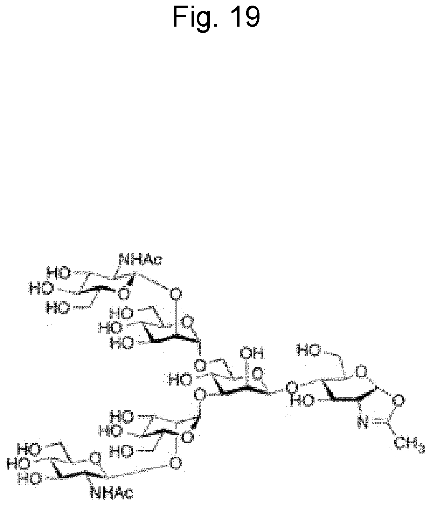

2-3-1. Into 98 µL of a 100 mM phosphate buffer solution (pH 6.5), Herceptin (2 mg; 200 µL) deglycosylated with ENGase, GO-Oxazoline (manufactured by Fushimi Pharmaceutical Co., Ltd.; 1 mM; 1000 µL, Figure 19; hereinafter, referred to as GO-Ox), and EndoS D233Q (4.35 µg; 2 µL) were added, and the mixture was reacted at 30°C for 12 hours. Antibodies in the solution thus reacted were purified by the same procedures as in 2-1-2.

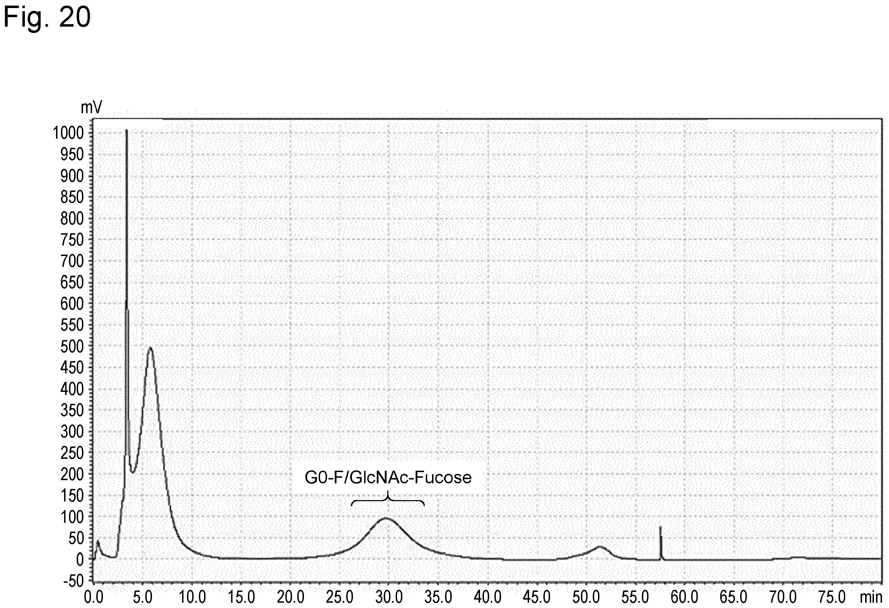

2-3-2. The Herceptin antibodies obtained by purification were fractionated using Tosoh TSKgel(R) FcR-IIIA-NPR. The fractionation results are shown in Figure 20. A peak around a retention time of 30 minutes (GO-F/GlcNAc-Fucose of Figure 20) was obtained by the fractionation.

Fractionation conditions
Apparatus: Shimadzu HPLC system Prominence
Column: Tosoh TSKgel(R) FcR-IIIA-NPR 4.6 mm I.D. × 10 cm, 5 µm
Column temperature: 25°C
Flow rate: 1 ml/min
Detection: UV 280 nm
Mobile phase A: 50 mM citrate buffer (pH 6.5)
Mobile phase B: 50 mM citrate buffer (pH 4.5)

### Gradient conditions

1. 0-2min A 100%
2. 2-45min A100-0%
3. 45-55min A 0%
4. 55-60min A 0-100%
5. 60-80min A 100%

2-3-3. The peak obtained by fractionation was subjected to intact mass analysis by LC-MS/MS.
Intact mass analysis
Apparatus: Waters Acquity H-Class Bio UHPLC System with Vion IMS Qtof
Column: Waters MassPREP Desalting Column
Column temperature: 80°C
Mobile phase: solution A: 0.1% formic acid
solution B: acetonitrile
Gradient conditions are shown in Table 3.

**[Table 3]**

| No. | Time (min) | Flow (ml/min) | A (%) | B (%) |
|---|---|---|---|---|
| 1 | 0 | 0.5 | 95 | 5 |
| 2 | 0.5 | 0.5 | 95 | 5 |
| 3 | 0.51 | 0.2 | 95 | 5 |
| 4 | 3 | 0.2 | 10 | 90 |
| 5 | 3.1 | 0.5 | 10 | 90 |
| 6 | 3.4 | 0.5 | 10 | 90 |
| 7 | 3.6 | 0.5 | 95 | 5 |
| 8 | 3.8 | 0.5 | 95 | 5 |

m/z range: 400-4000
Capillary voltage: 3.00 kV
Cone voltage: 150 V
Source temperature: 150°C
Desolvation temperature: 600°C
Deconvolution Software: Waters UNIFI software v1.8.2.

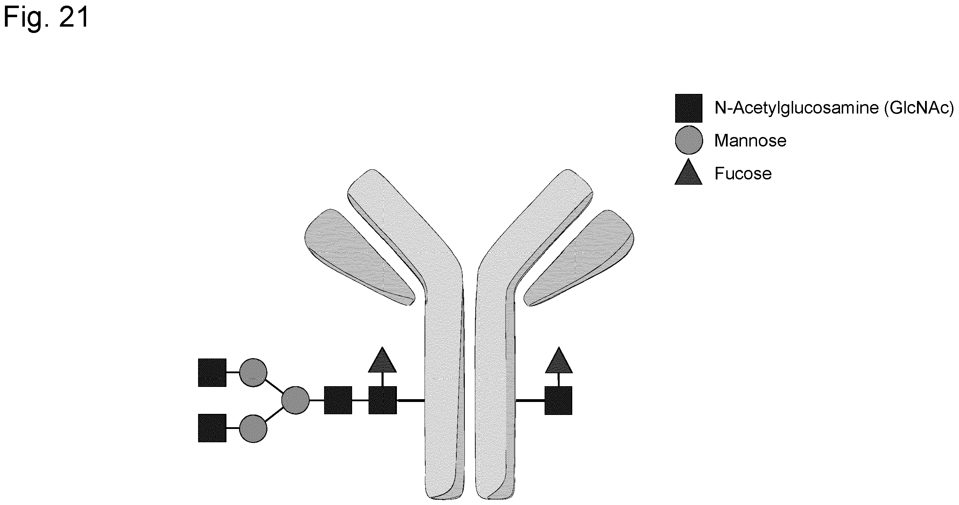

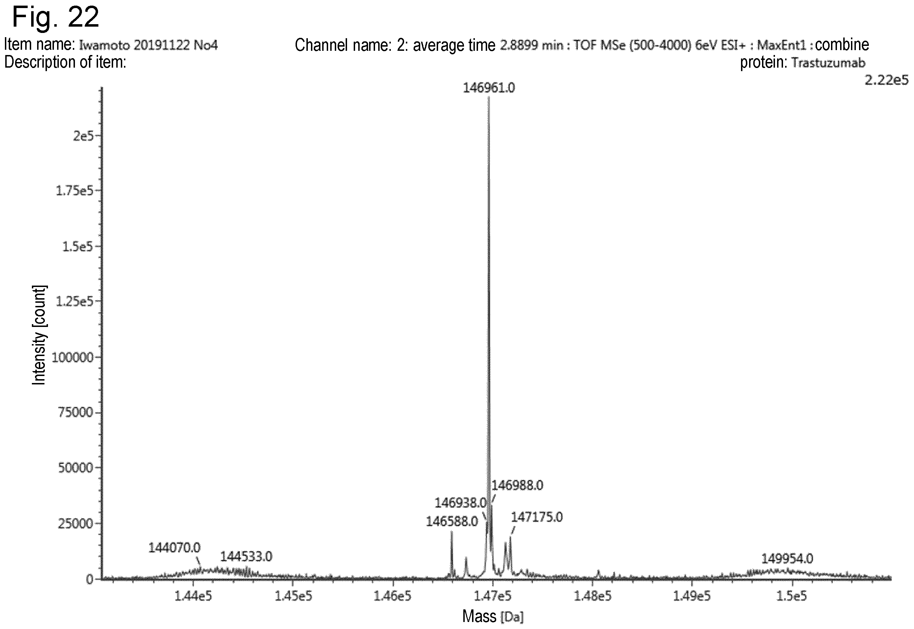

The detected mass was 146961, revealing a structure (Figure 21) having G0-F attached to one GlcNAc-Fucose site of Asn297 (Figure 22).

### 2-4. Preparation of Herceptin[M3-F/GlcNAc-Fucose]

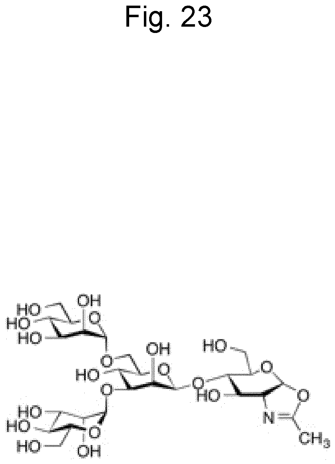

2-4-1. Into 98 µL of a 100 mM phosphate buffer solution (pH 6.5), Herceptin (2 mg; 200 µL) deglycosylated with ENGase, M3-Oxazoline (manufactured by Fushimi Pharmaceutical Co., Ltd.; 1 mM; 1000 µL, Figure 23; hereinafter, referred to as M3-Ox), and EndoS D233Q (4.35 µg; 2 µL) were added, and the mixture was reacted at 30°C for 12 hours. Antibodies in the solution thus reacted were purified by the same procedures as in 2-1-2.

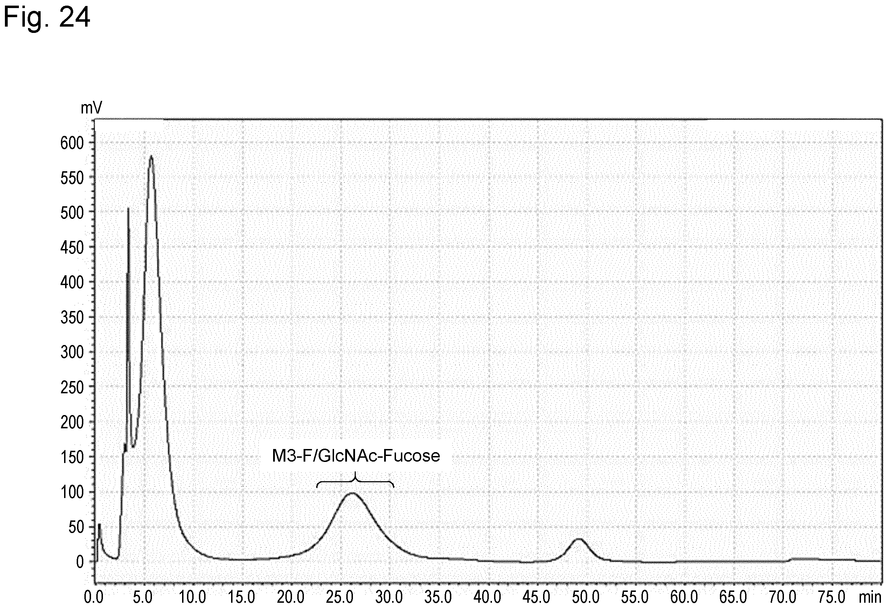

2-4-2. The Herceptin antibodies obtained by purification were fractionated using Tosoh TSKgel(R) FcR-IIIA-NPR. The fractionation results are shown in Figure 24. A peak around a retention time of 26 minutes (M3-F/GlcNAc-Fucose of Figure 24) was obtained by the fractionation.

Fractionation conditions
Apparatus: Shimadzu HPLC system Prominence
Column: Tosoh TSKgel(R) FcR-IIIA-NPR 4.6 mm I.D. × 10 cm, 5 µm
Column temperature: 25°C
Flow rate: 1 ml/min
Detection: UV 280 nm
Mobile phase A: 50 mM citrate buffer (pH 6.5)
Mobile phase B: 50 mM citrate buffer (pH 4.5)

### Gradient conditions

1. 0-2min A 100%
2. 2-45min A100-0%
3. 45-55min A 0%
4. 55-60min A 0-100%
5. 60-80min A 100%

2-4-3. The peak obtained by fractionation was subjected to intact mass analysis by LC-MS/MS.
Intact mass analysis
Apparatus: Waters Acquity H-Class Bio UHPLC System with Vion IMS Qtof
Column: Waters MassPREP Desalting Column
Column temperature: 80°C
Mobile phase: solution A: 0.1% formic acid
solution B: acetonitrile
Gradient conditions are shown in Table 4.

**[Table 4]**

| No. | Time (min) | Flow (ml/min) | A (%) | B (%) |
|---|---|---|---|---|
| 1 | 0 | 0.5 | 95 | 5 |
| 2 | 0.5 | 0.5 | 95 | 5 |
| 3 | 0.51 | 0.2 | 95 | 5 |
| 4 | 3 | 0.2 | 10 | 90 |
| 5 | 3.1 | 0.5 | 10 | 90 |
| 6 | 3.4 | 0.5 | 10 | 90 |
| 7 | 3.6 | 0.5 | 95 | 5 |
| 8 | 3.8 | 0.5 | 95 | 5 |

m/z range: 400-4000
Capillary voltage: 3.00 kV
Cone voltage: 150 V
Source temperature: 150°C
Desolvation temperature: 600°C
Deconvolution Software: Waters UNIFI software v1.8.2.

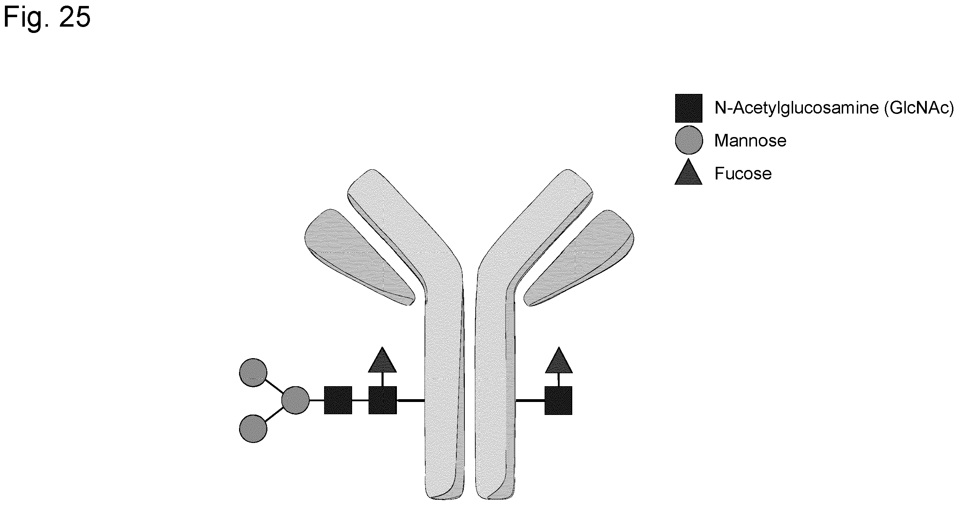

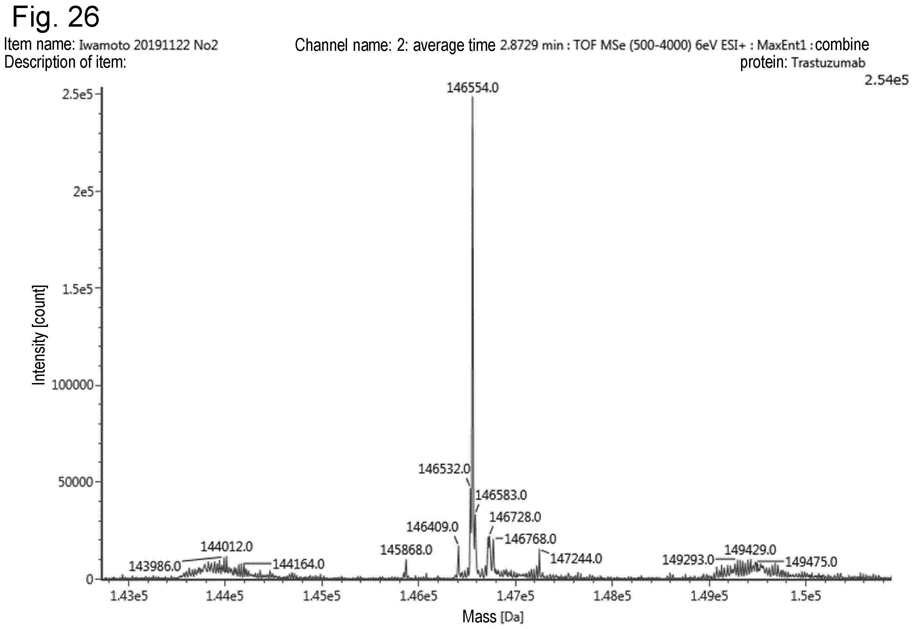

The detected mass was 146554, revealing a structure (Figure 25) having M3-F attached to one GlcNAc-Fucose site of Asn297 (Figure 26).

### [Example 3] Preparation of antibody having left-right asymmetric sugar chains

Antibodies each having different sugar chains on the left and the right (left-right asymmetric) were prepared using Herceptin[SG-F/GlcNAc-Fucose] having SG at one location and using G2-G2-Ox different from SG.

### 3-1. Preparation of Herceptin[SG-F/G2-F]

3-1-1. Into 98 µL of a 100 mM phosphate buffer solution (pH 6.5), Herceptin[SG-F/GlcNAc-Fucose] (2 mg; 200 µL) having SG at one location with ENGase, G2-Ox (manufactured by Fushimi Pharmaceutical Co., Ltd.; 1 mM; 100 µL), and EndoS D233Q (4.35 µg; 2 µL) were added, and the mixture was reacted at 30°C for 12 hours.

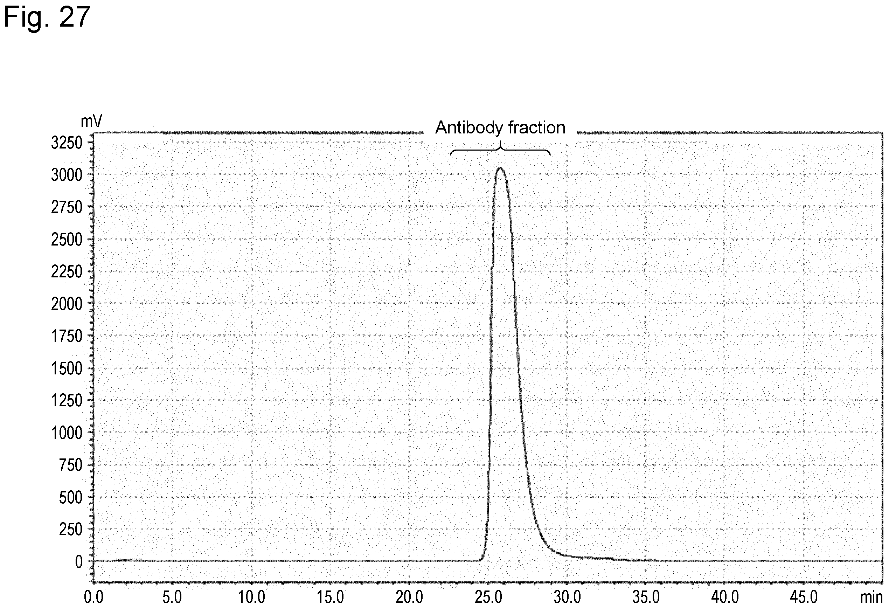

3-1-2. Antibodies were purified from the reaction solution with a Protein A column. A chromatogram obtained by the fractionation is shown in Figure 27. A peak around a retention time of 25 minutes (Antibody fraction of Figure 27) was recovered as antibodies.

Purification conditions
Apparatus: Shimadzu HPLC system
Column: Tosoh ToyoScreen AF-Protein A HC-650F 1 ml
Column temperature: 25°C
Flow rate: 1 ml/min
Detection: UV 280 nm
Mobile phase A: 0.1 M phosphate buffer solution (pH 6.5)
Mobile phase B: 0.1 M citrate buffer (pH 3.5)

### Gradient conditions

1. 0-15 min A 100%
2. 15-15.01 min A 100-0%
3. 15.01-25 min A 0%
4. 25-25.01 min A 0-100%
5. 25.01-50 min A 100%

The recovered fractions were concentrated by ultrafiltration with Sartorius Vivaspin Turbo 15 (50 K). Then, the buffer was replaced with a 50 mM citrate buffer (pH 6.5) (2,460 × g, 4°C).

3-1-3. The protein concentration of the obtained Herceptin solution was measured. NanoDrop(TM) 2000C manufactured by Thermo Fisher Scientific Inc. (attached software, mode; Protein A280, Type; IgG for measurement). The amount of recovery: 2 mg (1.8 mL, 1.1 mg/mL).

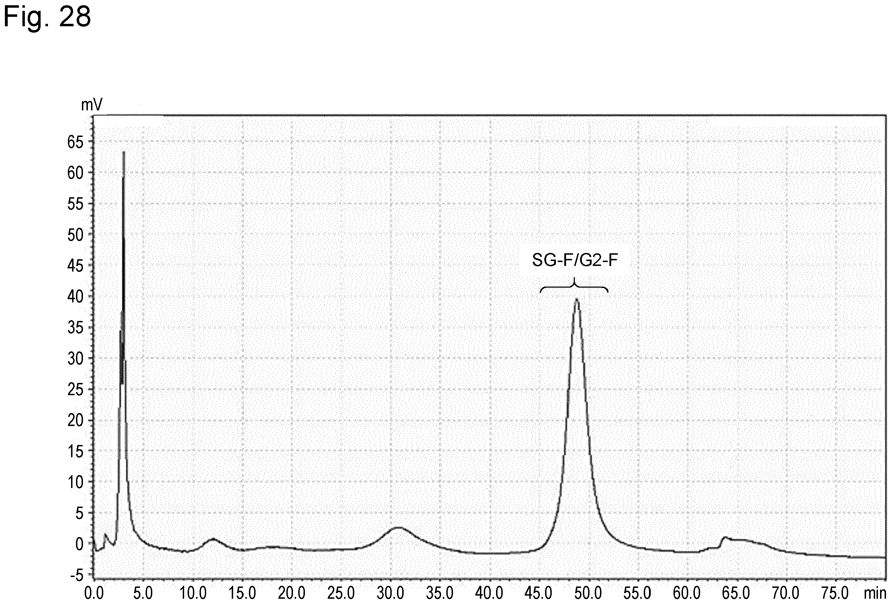

3-1-4. The Herceptin antibodies obtained by purification were fractionated using Tosoh TSKgel(R) FcR-IIIA-NPR. The fractionation results are shown in Figure 28. A peak around a retention time of 49 minutes (SG-F/G2-F of Figure 28) was obtained by the fractionation.

Fractionation conditions
Apparatus: Shimadzu HPLC system Prominence
Column: Tosoh TSKgel(R) FcR-IIIA-NPR 4.6 mm I.D. × 10 cm, 5 µm
Column temperature: 25°C
Flow rate: 1 ml/min
Detection: UV 280 nm
Mobile phase A: 50 mM citrate buffer (pH 6.5)
Mobile phase B: 50 mM citrate buffer (pH 4.5)

### Gradient conditions

1. 0-2min A 100%
2. 2-45min A100-0%
3. 45-55min A 0%
4. 55-60min A 0-100%
5. 60-80min A 100%

3-1-5. The peak obtained by fractionation was subjected to intact mass analysis by LC-MS/MS.
Intact mass analysis
Apparatus: Waters Acquity H-Class Bio UHPLC System with Vion IMS Qtof
Column: Waters MassPREP Desalting Column
Column temperature: 80°C
Mobile phase: solution A: 0.1% formic acid
solution B: acetonitrile
Gradient conditions are shown in Table 5.

**[Table 5]**

| No. | Time (min) | Flow (ml/min) | A (%) | B (%) |
|---|---|---|---|---|
| 1 | 0 | 0.5 | 95 | 5 |
| 2 | 0.5 | 0.5 | 95 | 5 |
| 3 | 0.51 | 0.2 | 95 | 5 |
| 4 | 3 | 0.2 | 10 | 90 |
| 5 | 3.1 | 0.5 | 10 | 90 |
| 6 | 3.4 | 0.5 | 10 | 90 |
| 7 | 3.6 | 0.5 | 95 | 5 |
| 8 | 3.8 | 0.5 | 95 | 5 |

m/z range: 400-4000
Capillary voltage: 3.00 kV
Cone voltage: 150 V
Source temperature: 150°C
Desolvation temperature: 600°C
Deconvolution Software: Waters UNIFI software v1.8.2.

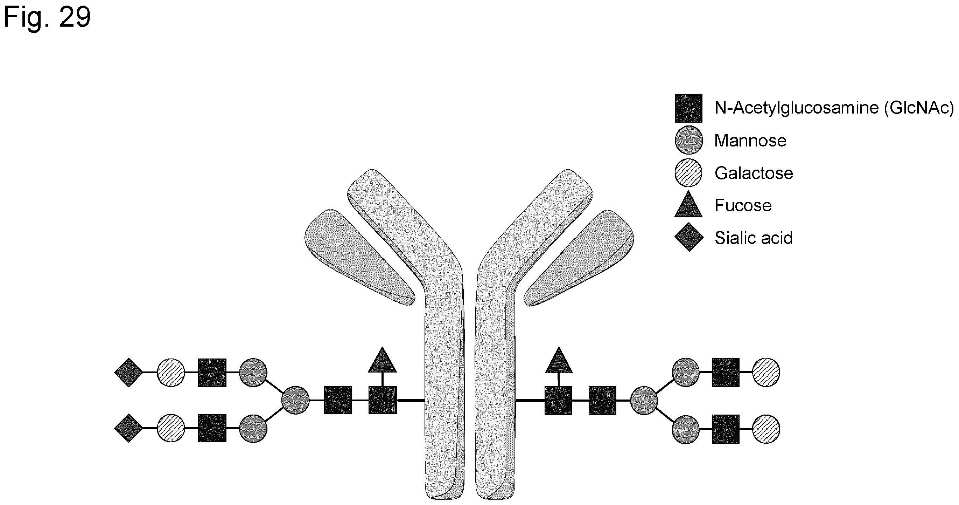

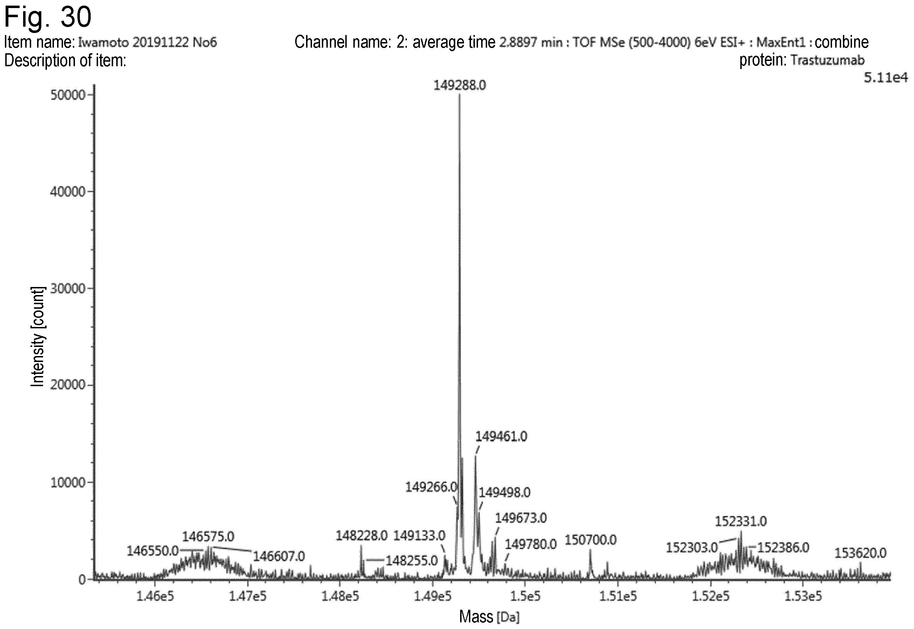

The detected mass was 149288, revealing a structure (Figure 29) having SG-F and G2-F attached to two GlcNAc-Fucose sites, respectively, of Asn297 (Figure 30).

### 3-2. Preparation of Herceptin[G0-F/SG-F]

3-2-1. Into 98 µL of a 100 mM phosphate buffer solution (pH 6.5), Herceptin[GO-F/GlcNAc-Fucose] (2 mg; 200 µL) having G0 at one location with ENGase, SG-Ox (manufactured by Fushimi Pharmaceutical Co., Ltd.; 1 mM; 100 µL), and EndoS D233Q (4.35 µg; 2 µL) were added, and the mixture was reacted at 30°C for 12 hours. Antibodies in the solution thus reacted were purified by the same procedures as in 3-1-2.

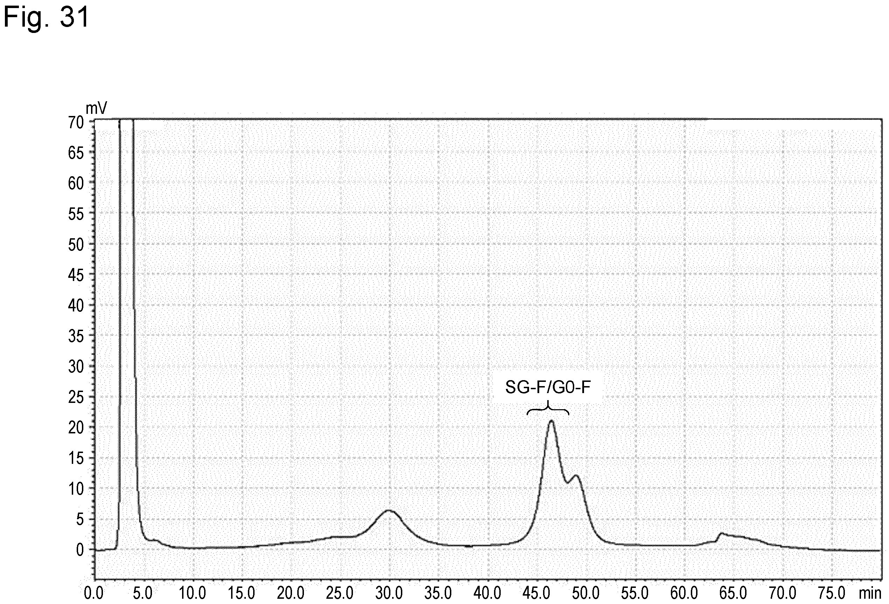

3-2-2. The Herceptin antibodies obtained by purification were fractionated using Tosoh TSKgel(R) FcR-IIIA-NPR. The fractionation results are shown in Figure 31. A peak around a retention time of 46 minutes (SG-F/G0-F of Figure 31) was obtained by the fractionation.

Fractionation conditions
Apparatus: Shimadzu HPLC system Prominence
Column: Tosoh TSKgel(R) FcR-IIIA-NPR 4.6 mm I.D. × 10 cm, 5 µm
Column temperature: 25°C
Flow rate: 1 ml/min
Detection: UV 280 nm
Mobile phase A: 50 mM citrate buffer (pH 6.5)
Mobile phase B: 50 mM citrate buffer (pH 4.5)

### Gradient conditions

1. 0-2min A 100%
2. 2-45min A100-0%
3. 45-55min A 0%
4. 55-60min A 0-100%
5. 60-80min A 100%

3-2-3. The peak obtained by fractionation was subjected to intact mass analysis by LC-MS/MS.
Intact mass analysis
Apparatus: Waters Acquity H-Class Bio UHPLC System with Vion IMS Qtof
Column: Waters MassPREP Desalting Column
Column temperature: 80°C
Mobile phase: solution A: 0.1% formic acid
solution B: acetonitrile
Gradient conditions are shown in Table 6.

**[Table 6]**

| No. | Time (min) | Flow (ml/min) | A (%) | B (%) |
|---|---|---|---|---|
| 1 | 0 | 0.5 | 95 | 5 |
| 2 | 0.5 | 0.5 | 95 | 5 |
| 3 | 0.51 | 0.2 | 95 | 5 |
| 4 | 3 | 0.2 | 10 | 90 |
| 5 | 3.1 | 0.5 | 10 | 90 |
| 6 | 3.4 | 0.5 | 10 | 90 |
| 7 | 3.6 | 0.5 | 95 | 5 |
| 8 | 3.8 | 0.5 | 95 | 5 |

m/z range: 400-4000
Capillary voltage: 3.00 kV
Cone voltage: 150 V
Source temperature: 150°C
Desolvation temperature: 600°C
Deconvolution Software: Waters UNIFI software v1.8.2.

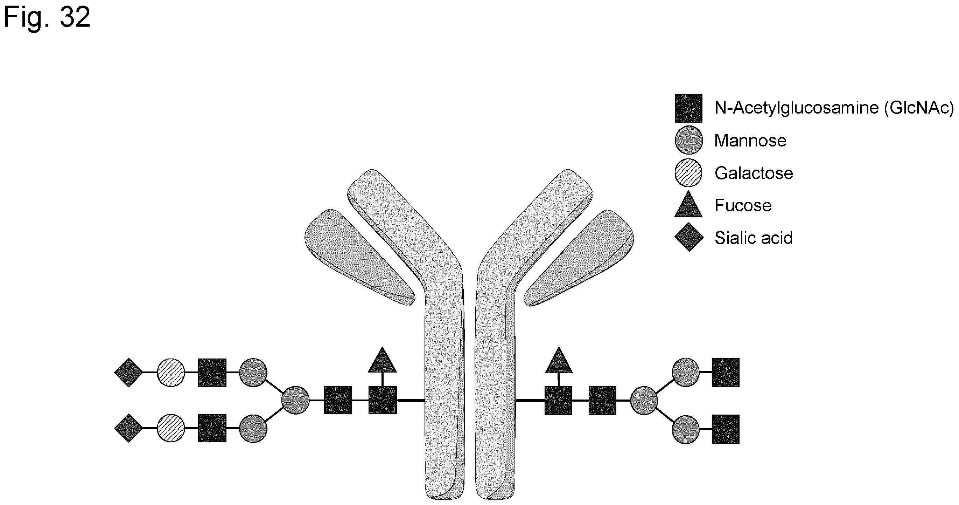

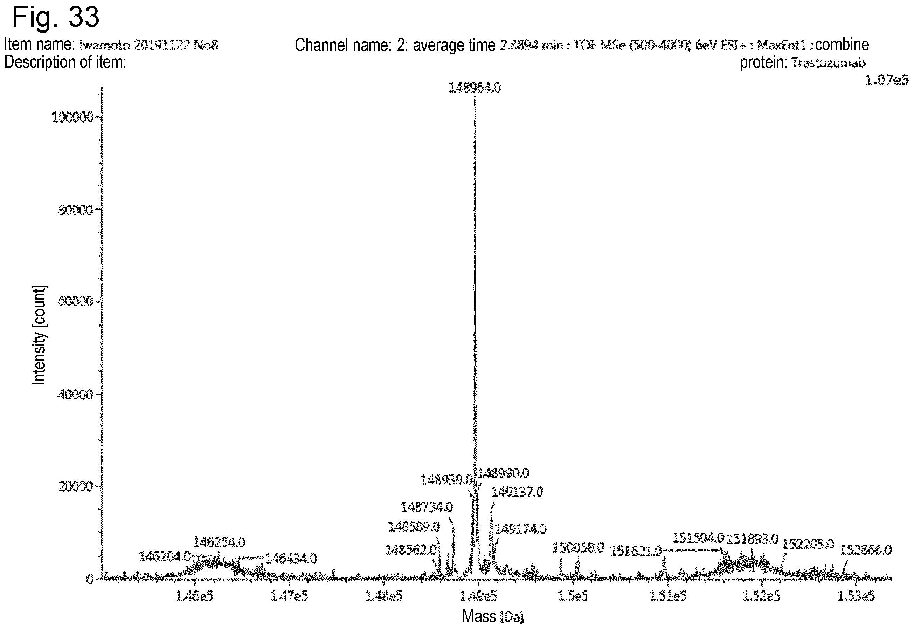

The detected mass was 148964, revealing a structure (Figure 32) having SG-F and G0-F attached to two GlcNAc-Fucose sites, respectively, of Asn297 (Figure 33).

### 3-3. Preparation of Herceptin[G2-F/M3-F]

3-3-1. Into 98 µL of a 100 mM phosphate buffer solution (pH 6.5), Herceptin[G2-F/GlcNAc-Fucose] (2 mg; 200 µL) having G2 at one location with ENGase, M3-Ox (manufactured by Fushimi Pharmaceutical Co., Ltd.; 1 mM; 100 µL), and EndoS D233Q (4.35 µg; 2 µL) were added, and the mixture was reacted at 30°C for 12 hours. Antibodies in the solution thus reacted were purified by the same procedures as in 3-1-2.

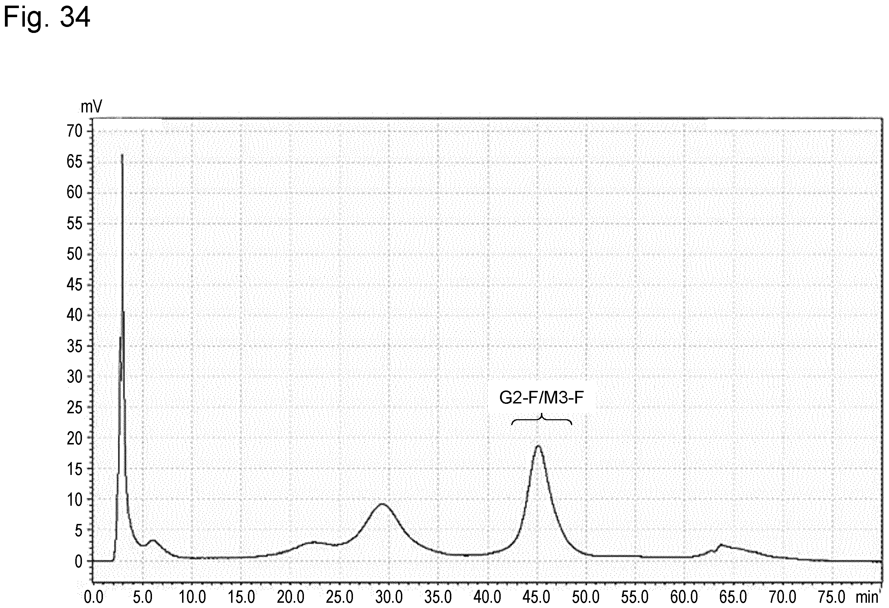

3-3-2. The Herceptin antibodies obtained by purification were fractionated using Tosoh TSKgel(R) FcR-IIIA-NPR. The fractionation results are shown in Figure 34. A peak around a retention time of 45 minutes (G2-F/M3-F of Figure 34) was obtained by the fractionation.

Fractionation conditions
Apparatus: Shimadzu HPLC system Prominence
Column: Tosoh TSKgel(R) FcR-IIIA-NPR 4.6 mm I.D. × 10 cm, 5 µm
Column temperature: 25°C
Flow rate: 1 ml/min
Detection: UV 280 nm
Mobile phase A: 50 mM citrate buffer (pH 6.5)
Mobile phase B: 50 mM citrate buffer (pH 4.5)

### Gradient conditions

1. 0-2min A 100%
2. 2-45min A100-0%
3. 45-55min A 0%
4. 55-60min A 0-100%
5. 60-80min A 100%

3-3-3. The peak obtained by fractionation was subjected to intact mass analysis by LC-MS/MS.
Intact mass analysis
Apparatus: Waters Acquity H-Class Bio UHPLC System with Vion IMS Qtof
Column: Waters MassPREP Desalting Column
Column temperature: 80°C
Mobile phase: solution A: 0.1% formic acid
solution B: acetonitrile
Gradient conditions are shown in Table 7.

**[Table 7]**

| No. | Time (min) | Flow (ml/min) | A (%) | B (%) |
|---|---|---|---|---|
| 1 | 0 | 0.5 | 95 | 5 |
| 2 | 0.5 | 0.5 | 95 | 5 |
| 3 | 0.51 | 0.2 | 95 | 5 |
| 4 | 3 | 0.2 | 10 | 90 |
| 5 | 3.1 | 0.5 | 10 | 90 |
| 6 | 3.4 | 0.5 | 10 | 90 |
| 7 | 3.6 | 0.5 | 95 | 5 |
| 8 | 3.8 | 0.5 | 95 | 5 |

m/z range: 400-4000
Capillary voltage: 3.00 kV
Cone voltage: 150 V
Source temperature: 150°C
Desolvation temperature: 600°C
Deconvolution Software: Waters UNIFI software v1.8.2.

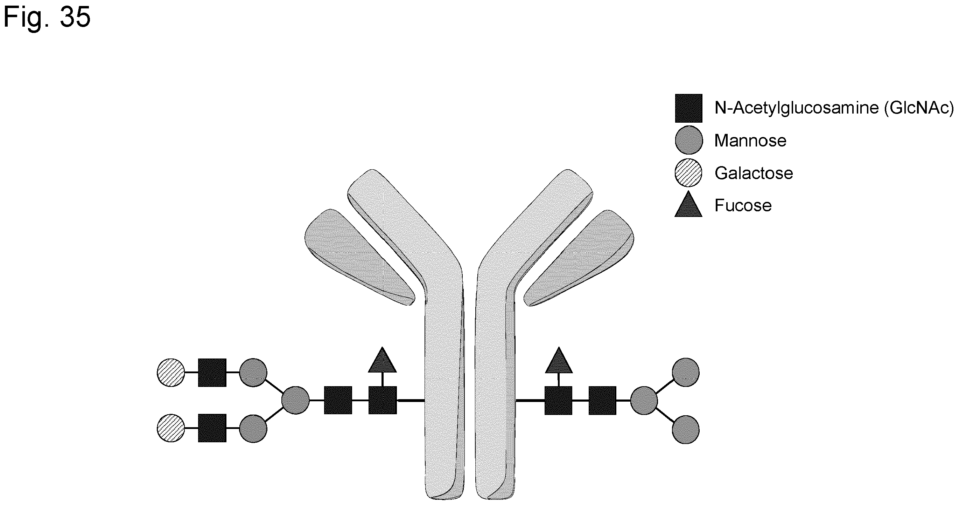

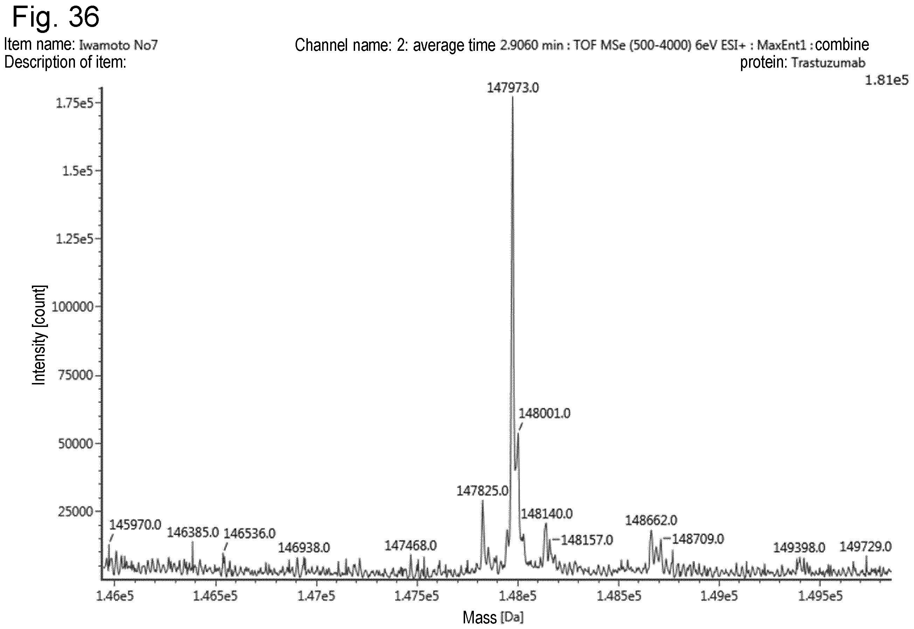

The detected mass was 147975, revealing a structure (Figure 35) having G0-F and M3-F attached to two GlcNAc-Fucose sites, respectively, of Asn297 (Figure 36).

Thus, use of an antibody having one sugar chain obtained by fractionation with Tosoh TSKgel(R) FcR-IIIA-NPR enables an antibody having asymmetric sugar chains to be prepared by introducing a sugar chain different therefrom as the second sugar chain.

### Industrial Applicability

A population of antibodies comprising homogeneous antibodies each having left-right asymmetric sugar chains can be used as antibody drugs, etc. The population of antibodies can be used as standards for antibody drug quality control.

All publications, patents and patent applications cited herein are incorporated herein by reference in their entirety.

## Claims

1. A population of antibodies comprising homogeneous antibodies in which N-linked complex sugar chains attached to asparagine (Asn) at position 297 of CH domains of Fc regions of two heavy chains on the left and the right of each antibody are sugar chains structurally different from each other.

2. The population of antibodies according to claim 1, wherein the population comprises 90% or more of the antibodies in which the N-linked complex sugar chains attached to asparagine (Asn) at position 297 of CH domains of Fc regions of two heavy chains on the left and the right of each antibody are different from each other.

3. The population of antibodies according to claim 1 or 2, wherein N-acetylglucosamine (GlcNAc) at reducing ends of the N-linked complex sugar chains attached to asparagine (Asn) at position 297 located in the CH domains of the antibody Fc regions is fucosylated.

4. A method for producing a population of antibodies comprising homogeneous antibodies in which N-linked complex sugar chains attached to asparagine (Asn) at position 297 of CH domains of Fc regions of two heavy chains on the left and the right of each antibody are sugar chains structurally different from each other, the method comprising the steps of:
(i) cleaving sugar chains attached to two heavy chains on the left and the right of each antibody in an antibody composition with endo-β-N-acetylglucosaminidase (ENGase), and purifying and isolating antibodies from which the sugar chains of both the heavy chains have been cleaved;
(ii) mixing a population of the antibodies from which the sugar chains of both the heavy chains have been cleaved, obtained in the step (i), any sugar chain X in an oxazoline form or an oxazoline-generating sugar derivative, the sugar chain of which is sugar chain X, and ENGase modified so as to suppress sugar chain cleavage activity and improve sugar chain transfer activity, to prepare antibodies in which the sugar chain X is attached to only one of the two heavy chains on the left and the right, followed by purification and isolation of the antibodies; and
(iii) mixing the antibodies in which the sugar chain X is attached to only one of the two heavy chains on the left and the right, obtained in the step (ii), sugar chain Y in an oxazoline form which is structurally different from the sugar chain X, or an oxazoline-generating sugar derivative, the sugar chain of which is sugar chain Y, and ENGase modified so as to suppress sugar chain cleavage activity and improve sugar chain transfer activity, to prepare antibodies in which the sugar chain Y is attached to the other heavy chain of the two heavy chains on the left and the right and in which the sugar chains attached to the two heavy chains on the left and the right are sugar chains structurally different from each other, followed by purification and isolation of the antibodies.

5. The method according to claim 4, wherein the antibodies are purified and isolated by use of affinity chromatography using a carrier with an Fcγ receptor immobilized thereon as a ligand.

6. The method according to claim 4 or 5, wherein the ENGase is EndoS.

7. The method according to any one of claims 4 to 6, wherein the ENGase modified so as to suppress sugar chain cleavage activity and improve sugar chain transfer activity is selected from the group consisting of EndoS D233Q, Endo S2 D184M, Endo S2 D184Q, EndoS D233Q/Q303L, D233Q/A303L/E350Q, and Endo M N175Q.

8. The method according to any one of claims 4 to 7, wherein a molecular weight of the sugar chain X to be attached in the step (ii) is larger than a molecular weight of the sugar chain Y to be attached in the step (iii).

9. The method according to any one of claims 4 to 8, wherein the produced population of antibodies comprises 90% or more of the antibodies in which the N-linked complex sugar chains attached to asparagine (Asn) at position 297 of CH domains of Fc regions of two heavy chains on the left and the right of each antibody are different from each other.

10. The method according to any one of claims 4 to 9, wherein N-acetylglucosamine (GlcNAc) at reducing ends of the N-linked complex sugar chains attached to asparagine (Asn) at position 297 located in the CH domains of the antibody Fc regions is fucosylated in the produced population of antibodies.

11. A method for fractionating antibodies in a population of antibodies into a non-glycosylated antibody, an antibody glycosylated at one heavy chain, and an antibody glycosylated at two heavy chains by use of affinity chromatography using a support with an Fcγ receptor immobilized thereon as a ligand.

12. The method according to claim 11, wherein the population of antibodies is treated with endo-P-N-acetylglucosaminidase (ENGase), and then, the antibodies in the population of antibodies are fractionated into a non-glycosylated antibody, an antibody glycosylated at one heavy chain, and an antibody glycosylated at two heavy chains by use of affinity chromatography using a carrier with an Fcγ receptor immobilized thereon as a ligand.
